# EUROPEAN PATENT APPLICATION

(11) **EP 4 603 490 A1**
(43) Date of publication of application: **20.08.2025**
(21) Application number: 23877322.0
(22) Date of filing: 12.10.2023
(51) Int. Cl.: C07D 453/02, C08G 61/10, H01B 1/06, C07D 295/073, H01M 8/10, H01M 8/1023, C25B 13/08

(54) **COMPOUND, POLYMER, ELECTROLYTE MEMBRANE, FUEL CELL AND ELECTROLYSIS APPARATUS**

(30) Priority: 13.10.2022 JP 2022164813
(71) Applicant: INSTITUTE OF SCIENCE TOKYO, Tokyo 152-8550 (JP); Nippon Kayaku Kabushiki Kaisha, Tokyo 100-0005 (JP)
(72) Inventor: MIYANISHI Shoji, Tokyo 152-8550 (JP); YAMAGUCHI Takeo, Tokyo 152-8550 (JP)
(74) Representative: INNOV-GROUP
(86) International application number: PCT/JP2023/036954
(87) International publication number: WO 2024/080321

(57) **Abstract**

The purpose of the present invention is to provide a compound capable of producing a polymer having excellent alkali durability, a polymer including the compound as a monomer, an electrolyte membrane having excellent alkali durability using the polymer, and a fuel cell and an electrolysis apparatus using the electrolyte membrane. A compound represented by the following Formula (1) and a polymer including the compound as a monomer.

(X¹-)₂Ar¹(-L¹-R^{N+})ₙ(A^{c-})_{n/c} ··· (1)

Where, X¹ is a hydrogen atom or a halogen atom, Ar¹ is a 2+n valent group having an aromatic ring, L¹ is a single bond or divalent hydrocarbon group, R^{N+} is a group having a ring structure containing N⁺, A^{C-} is a c valent counter anion, n is an integer of 1 or more, c is 1 or 2.

## Description

### Technical Field

The present disclosure relates to compounds, polymers, electrolyte membranes, fuel cells, and electrolysis apparatuses.

### Background Art

Electrolyte membranes and electrolyte ionomers are used in various fuel cells, such as solid polymer electrolyte fuel cells and solid alkaline fuel cells, and in various electrolysis techniques, such as water electrolysis. The electrolyte membranes are required to have excellent ionic conductivity as well as chemical and mechanical durability to withstand long-term use. In addition, the electrolyte ionomers are required to have high chemical durability and high fuel gas permeability.

As a proton-conducting material for electrolyte membranes with high swelling resistance and a high density of ion-exchange groups for high proton conductivity, the present inventors have disclosed, in Patent Literature 1, a proton-conducting material including repeating units having a specific hydrophilic moiety and a specific hydrophobic moiety and containing a specific cyclic compound in at least one of the hydrophilic moiety and the hydrophobic moiety. The proton-conducting material has a structure in which the hydrophilic moiety and the hydrophobic moiety are bonded via an ether bond.

The proton-conducting material with ether bonds in its main chain has the problem of degradation, particularly due to cleavage of the ether bonds under an alkaline environment.

The present inventors have disclosed a polymer that does not contain either bonds in the main chain and its manufacturing method in Patent Literature 2. The electrolyte membrane using the polymer of the Patent Literature 2 has excellent chemical durability and membrane strength. On the other hand, the present inventors have been conducting studies to further improve durability.

### Citation List

### Patent Literature

Patent Literature 1: Japanese Unexamined Patent Application Publication No. 2016-44242
Patent Literature 2: Japanese Unexamined Patent Application Publication No. 2021-42351

### Summary of Invention

### Technical Problem

The present disclosure aims to provide a compound capable of producing a polymer having excellent alkali durability, a polymer including the compound as a monomer, an electrolyte membrane having excellent alkali durability using the polymer, and a fuel cell and an electrolysis apparatus using the electrolyte membrane.

### Solution to Problem

The compound of the present disclosure is a compound represented by the Formula (1).

(X¹-)₂Ar¹(-L¹-R^{N+})ₙ(A^{c-})_{n/c} ··· (1)

Where X¹ is a hydrogen atom or a halogen atom,
Ar¹ is a 2+n valent group having an aromatic ring,
L¹ is a single bond or divalent hydrocarbon group,
R^{N+} is a group having a ring structure containing N⁺,
A^{c-} is a c valent counter anion,
n is an integer of 1 or more, and
c is 1 or 2.

In the one embodiment of the compound, L¹ is an alkylene group having 1 to 20 carbon atoms.

In the one embodiment of the compound, R^{N+} is a group represented by the following Formulas (N1) to (N8). 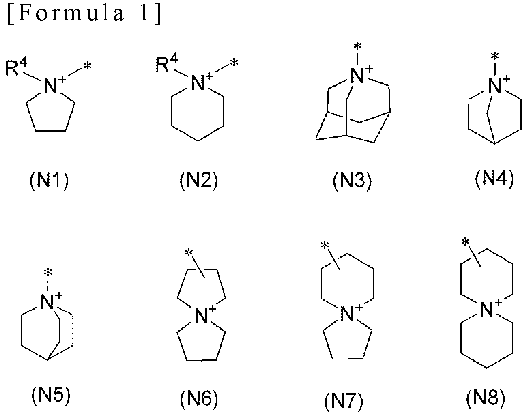 Where, R⁴ is an alkyl group having 1 to 6 carbon atoms,
* represents a bonding hand with L¹.

In the one embodiment of the compound, the (X¹-)₂Ar¹(-L¹-R^{N+})ₙ is represented by the following Formulas (a1) to (a12). 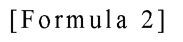 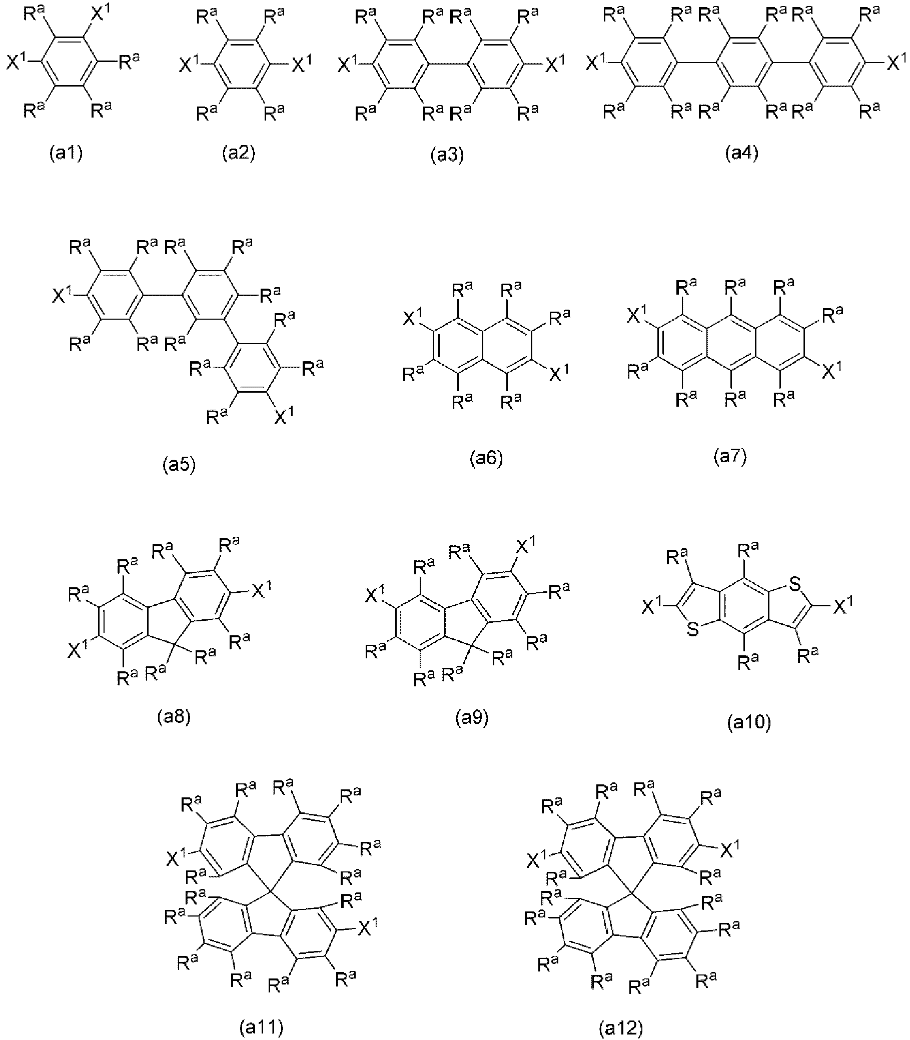 Where, R^{a} is a hydrogen atom, a group having no ionic functional group, or L¹-R^{N+}, and at least one of R^{a} is L¹-R^{N+}.

The polymer of the present disclosure is a polymer having a structural unit represented by the following Formula (2). 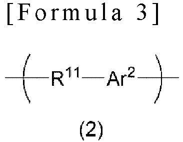 Where, Ar² is a divalent group having an aromatic ring,
R¹¹ is a group represented by the following Formula (11),

   (*-)₂Ar¹(-L¹-R^{N+})ₙ(A^{c-})_{n/c} ··· (11)
Ar¹ is a 2+n valent group having an aromatic ring,
L¹ is a single bond or divalent hydrocarbon group,
R^{N+} is a group having a ring structure containing N⁺,
A^{c-} is a c valent counter anion,
n is an integer of 1 or more,
c is 1 or 2, and
* represents a bonding hand with Ar².

In the one embodiment of the polymer, the (*-)₂Ar¹(-L¹-R^{N+})ₙ is represented by the following Formulas (a21) to (a32). 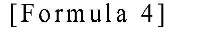 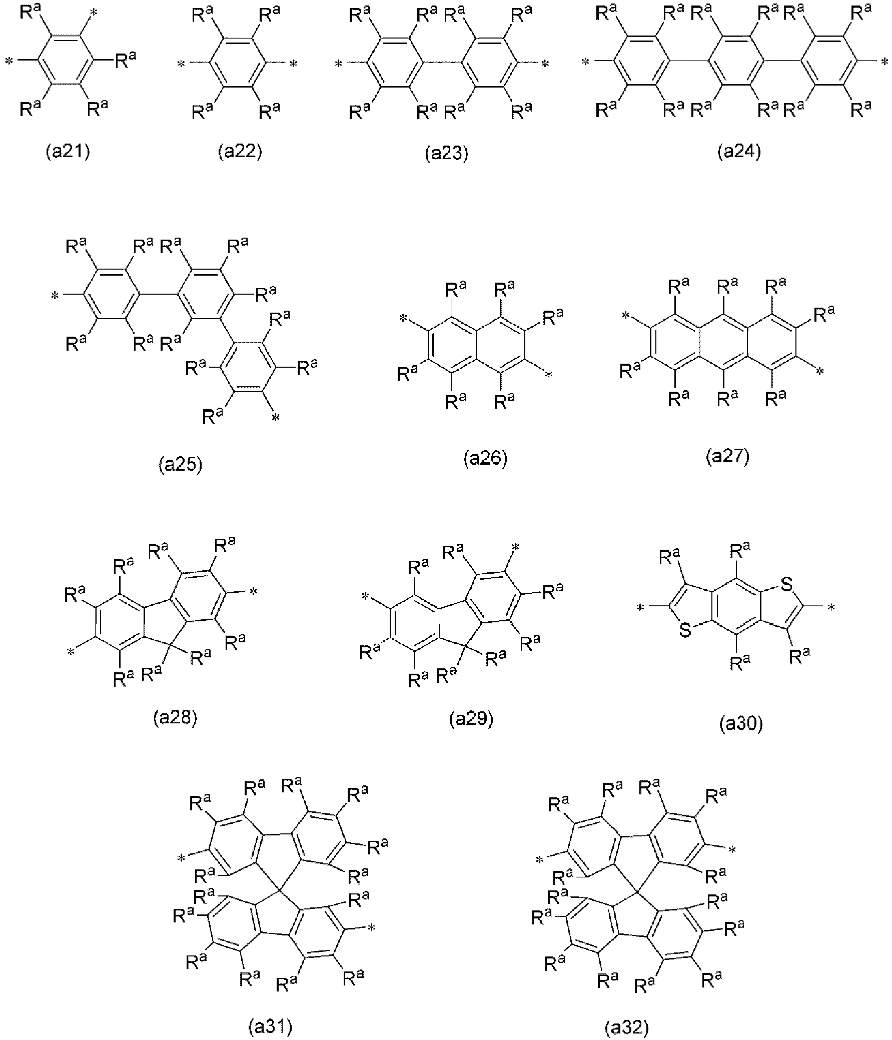 Where, R^{a} is a hydrogen atom, a group having no ionic functional group, or L¹-R^{N+}, and at least one of R^{a} is L¹-R^{N+}, and
* represents a bonding hand with Ar².

The present disclosure provides an electrolyte membrane including the above polymer.

In addition, the present disclosure provides a fuel cell and an electrolysis apparatus each including the above electrolyte membrane.

### Advantageous Effects of Invention

The present disclosure enables the provision of a compound capable of producing a polymer having excellent alkali durability, a polymer including the compound as a monomer, an electrolyte membrane having excellent alkali durability using the polymer, and a fuel cell and an electrolysis apparatus using the electrolyte membrane.

### Brief Description of Drawings

Fig. 1 is the ¹H-NMR spectrum of the compound i;
Fig. 2 is the ¹H-NMR spectrum of the compound ii;
Fig. 3 is the ¹H-NMR spectrum of Qin;
Fig. 4 is the ¹H-NMR spectrum of TMA;
Fig. 5 is the ¹H-NMR spectrum of TEA;
Fig. 6 is the ¹H-NMR spectrum of DiPrM;
Fig. 7 is the ¹H-NMR spectrum of Pyr;
Fig. 8 is the ¹H-NMR spectrum of Pep;
Fig. 9 is the ¹H-NMR spectrum of DMOc;
Fig. 10 is the ¹H-NMR spectrum of the polymer I;
Fig. 11 is the ¹H-NMR spectrum of the polymer II;
Fig. 12 is the ¹H-NMR spectrum of the polymer III;
Fig. 13 is a graph illustrating the test results of the alkali durability of the compound;
Fig. 14 is a graph illustrating the test results of the alkali durability of the electrolyte membrane;
Fig. 15 is the ¹H-NMR spectrum of the Pep-C6-H;
Fig. 16 is the ¹H-NMR spectrum of the Pyr-C6-H;
Fig. 17 is the ¹H-NMR spectrum of the QIN-C6-H;
Fig. 18 is the ¹H-NMR spectrum of the Pep-C10-H;
Fig. 19 is the ¹H-NMR spectrum of the Pyr-C10-H; and
Fig. 20 is the ¹H-NMR spectrum of the QIN-C10-H.

### Description of Embodiments

Hereinafter, the compound, polymer, electrolyte membrane, fuel cell, and electrolytic apparatus of the present disclosure will be described.

The term "polymer(s)" as used herein encompasses "copolymer(s)," unless otherwise specified.

The term "ionic functional group(s)" as used herein refers to a functional group(s) having dissociation properties and capable of ion exchange.

The symbol "-" indicating a numerical range as used herein means that the numerical values described before and after it are included as the lower and upper limits.

As used herein, the "compound represented by Formula (1)" may be referred to as "compound (1)". The same also applies to compounds, substituents and the like represented by other formulas.

As used herein, the "structural unit represented by Formula (2)" may be referred to as "structural unit (2)". The same also applies to structural units represented by other formulas.

In addition, when the same symbol appears in a one formula, it is not limited to representing the same substituent, and may represent different substituents with respect to each other within the range defined by the symbol.

### 1. Compound

The compound of the present disclosure is a compound represented by the Formula (1).

(X¹-)₂Ar¹(-L¹-R^{N+})ₙ(A^{c-})_{n/c} ··· (1)

Where, X¹ is a hydrogen atom or a halogen atom,
Ar¹ is a 2+n valent group having an aromatic ring,
L¹ is a single bond or divalent hydrocarbon group,
R^{N+} is a group having a ring structure containing N⁺,
A^{c-} is a c valent counter anion,
n is an integer of 1 or more, and
c is 1 or 2.

The compound (1) has the N⁺, which constitutes a quaternary ammonium group as an ionic functional group, forming a ring structure. This significantly improves the durability in relatively high temperature alkaline aqueous solutions, e.g. around 80°C. Therefore, the electrolyte membrane including a polymer that containing compound (1) as a monomer is suppressed from degradation even at high temperatures during operation of the fuel cell or the electrolytic apparatus.

Ar¹ is a 2+n valent group containing an aromatic ring. The aromatic ring in Ar¹ may be a benzene ring, as well as a condensed ring such as a naphthalene ring or an anthracene ring, and it may also be a heterocyclic ring containing oxygen (O), nitrogen (N), or sulfur (S) atoms (for example, thiophene and the like), and furthermore, it may be a group where multiple aromatic rings are linked via a single bond or a linking group (an aromatic ring-containing group). Examples of the structure in which multiple rings are linked by single bonds include biphenyl, terphenyl, fluorene, and the like. In addition, examples of the linking group include a spiro atom or a linear or branched alkylene group that may have a double bond. Specific examples of the alkylene group include -CH₂-, -CH(-)₂, -CH=CH-, and the like, where the H in the specific examples may be substituted with a group that does not have an ionic functional group, L¹-R^{N+} or the like, as described below. In addition, as Ar¹ having a spiro atom as a linking group, structures represented by formulas (a11) and (a12) or the like, which will be described below can be exemplified. Examples of the group in which multiple aromatic rings are linked through a linking group include diphenylmethane, triphenylmethane, stilbene, and the like. From the point of view of the durability of the polymer obtained, Ar¹ is preferably a group consisting of an aromatic ring or a group in which multiple aromatic rings are linked via a single bond, and more preferably, a 2+n valent residue of benzene, naphthalene, anthracene, biphenyl, terphenyl, or fluorene. The aromatic ring in Ar¹ may have substituents other than X¹ and L¹-R^{N+}, which will be described below. Examples of such substituents include groups that do not have ionic functional groups and the like in R^{a}, which will be described below. Among the R^{a} described below, the groups that do not have a hydrogen atom and do not have ionic functional group are included in Ar¹.

X¹ is a halogen atom (halogeno group) or a hydrogen atom. Examples of the halogen atom include F, Cl, Br, and I. From the viewpoint of facilitating the synthesis of the polymers described below, Br (bromo group) or I (iodo group) is preferred for X¹, with bromo group being more preferred. In addition, although the two X¹ in the Formula (1) may be the same or different, X¹ is preferably the same from the viewpoint of facilitating the synthesis of the polymer.

When X¹ is a halogen atom, it is preferably directly bonded to the carbon atom constituting the aromatic ring of Ar¹. Since X¹ is bonded to the carbon atom constituting the aromatic ring of Ar¹, the polymer described below has the carbon atom of the aromatic ring of Ar¹ directly bonded to the carbon atom of the aromatic ring of Ar², resulting in excellent polymer durability.

L¹ is a single bond or divalent hydrocarbon group. When L¹ is a single bond, Ar¹ and R^{N+} are directly bonded. When L¹ is a divalent hydrocarbon group, Ar¹ and R^{N+} are linked via L¹. As the divalent hydrocarbon group, a linear or branched alkylene group is preferable, among which a linear alkylene group is preferable, and an alkylene group having 1 to 20 carbon atoms is more preferable. The number of carbons of the alkylene group can be appropriately adjusted according to the physical properties required for the obtained polymer. For example, the ion-exchange group capacity of the polymer is increased by setting the carbon number of the alkylene group to 20 or less, preferably 16 or less, more preferably 12 or less. On the other hand, a polymer excellent in solubility and swelling resistance is obtained by setting the carbon number of the alkylene group to 2 or more, preferably 4 or more, more preferably 6 or more. In addition, from the viewpoint of alkali durability, it is preferable that L¹ is bonded to the carbon atom constituting Ar¹ without via a hetero element such as ether.

R^{N+} is a group having a ring structure containing N⁺ and is a quaternary ammonium group. By having R^{N+}, the obtained polymer has anion conductivity and has excellent alkali durability. Examples of R^{N+} include groups represented by the following Formula (3).

*-R¹N⁺R²R³R⁴ ··· (3)

Where, R¹ is a single bond or a group that is linked with R⁴ to form a ring structure containing N⁺,
R² is an alkyl group or a group that is linked with R³ or R³ and R⁴ to form a ring structure containing N⁺,
R³ is an alkyl group or a group that is linked with R² or R² and R⁴ to form a ring structure containing N⁺,
R⁴ is an alkyl group or a group that is linked with R¹ or R² and R³ to form a ring structure containing N⁺,
* represents a bonding hand with L¹ or Ar¹,
when R¹ is a single bond, R² and R³, or R², R³, and R⁴, are linked to form a ring structure containing N⁺.

When R¹ is a single bond, N⁺ is directly bonded to L¹ or Ar¹.

When R¹ and R⁴ are linked to form a ring structure containing N⁺, R^{N+} has a ring structure at the L¹ side, for example, as shown in the Formulas (N6) to (N8) described below. When R¹ and R⁴ are linked, the R¹-R⁴ is preferably an alkylene group having 4 or 5 carbon atoms. Where, the alkylene group has a bonding hand that binds to L¹.

When R² and R³ are linked to form a ring structure containing N⁺, R^{N+} has a ring structure at the terminal side, for example, as shown in the Formulas (N1), (N2), (N6) to (N8) described below. When R² and R³ are linked, the R²-R³ is preferably an alkylene group having 4 or 5 carbon atoms.

When R², R³ and R⁴ are linked to form a ring structure containing N⁺, R^{N+} has a bridged ring structure at the terminal side, for example, as shown in the Formulas (N3) to (N5) described below. The bridged ring is preferably a hydrocarbon except for N⁺. The carbon number of the bridged ring is preferably 6 to 18, and 6 to 12 is more preferable.

When R², R³, or R⁴ is an alkyl group, the alkyl group preferably has a carbon number of 1 to 6, and more preferably 1 to 3.

From the viewpoint of the durability of the compound and the polymer described below, it is preferable that R² and R³ are linked to form a ring structure containing N⁺, and it is more preferable that R^{N+} is a group represented by the following Formulas (N1) to (N8). 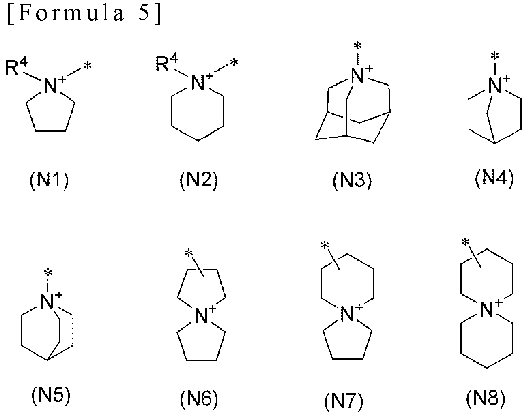 Where, R⁴ is an alkyl group having 1 or 6 carbon atoms,
* represents a bonding hand with L¹.

The number of R^{N+} (i.e., n) in compound (1) may be 1 or more, and from the viewpoint of ionic conductivity and polymer stability, 1 to 2 is preferred.

The aromatic ring of Ar¹ may have other substituent other than X¹ and L¹-R^{N+}. Preferably, the other substituent is a group that does not have an ionic functional group. Examples of the group that does not have an ionic functional group include an alkyl group having 1 to 20 carbon atoms that may have a substituent, a phenyl group that may have a substituent, and the like. As used herein, an ionic functional group represents a substituent that can exhibit ion exchangeability, and represents a sulfo group, carboxyl group, phosphate group (-HPO₃), and a quaternary ammonium group.

Specific examples of the above alkyl group include alkyl group such as a methyl group, an ethyl group, a propyl group, an n-butyl group, a tert-butyl group, a pentyl group, a hexyl group, and an octyl group, etc. and may have a phenyl group, etc. as a substituent. In addition, the substituent that the phenyl group may have include an alkyl group having 1 to 6 carbon atoms.

The A^{c-} is a monovalent or divalent counter anion. A^{c-} is not particularly limited, but a monovalent anion is preferred and an inorganic anion is preferred. The inorganic anion includes a chloride ion (Cl⁻), a bromide ion (Br⁻), an iodide ion (I⁻), a bicarbonate ion (HCO₃⁻), a carbonate ion (CO₃²⁻), a hydroxide ion (OH⁻), and the like.

Preferred structures of (X¹-)₂Ar¹(-L¹-R^{N+})ₙ of compound (1) include those represented by the following Formulas (a1) to (a12). 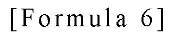 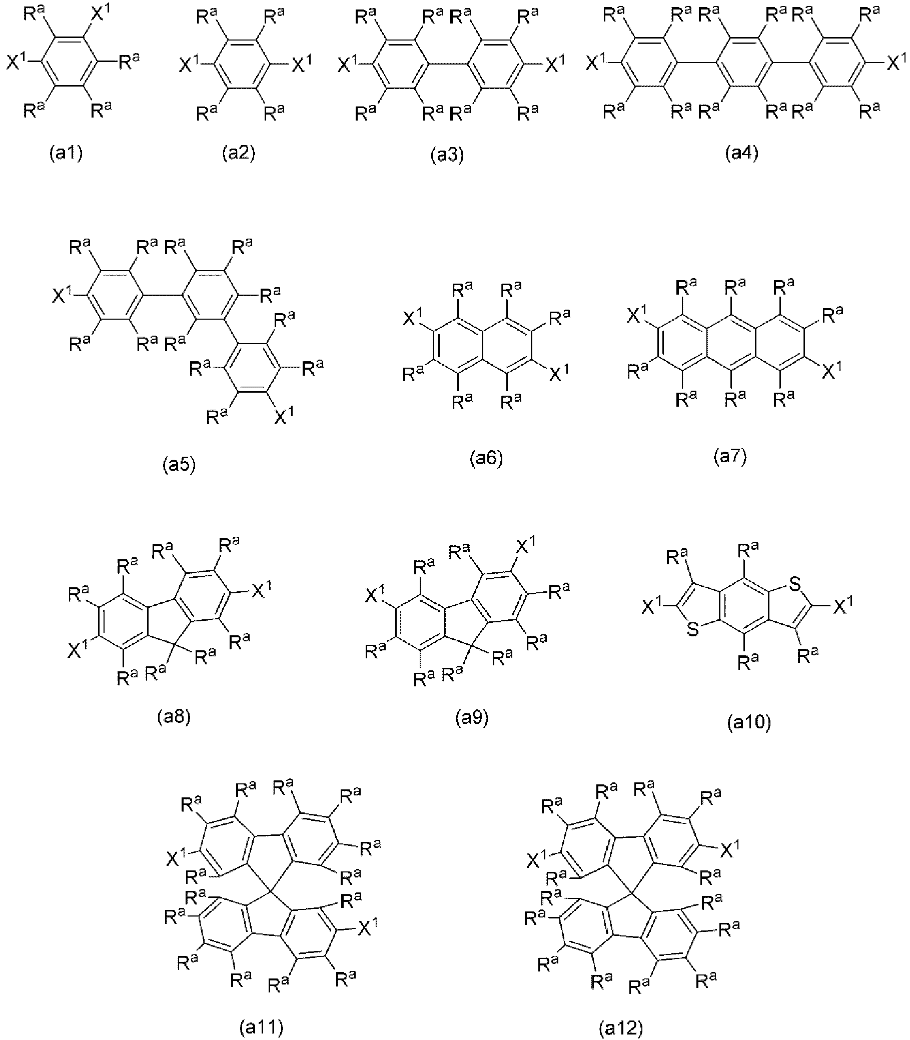 Where, R^{a} is a hydrogen atom, a group having no ionic functional group, or L¹-R^{N+}, and at least one of R^{a} is L¹-R^{N+}.

The method for synthesizing the compound represented by Formula (1) is not particularly limited, but for example, a halide including Ar¹ and an amine having a desired ring structure can be prepared and obtained by referring to the examples described below and the synthesis method described in Japanese Unexamined Patent Application Publication No. 2020-169287 and Patent Application Publication No. 2021-042351.Japanese Unexamined

### 2. Polymer

The polymer of the present disclosure has a structural unit represented by the following Formula (2). 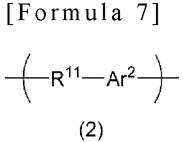 Where, Ar² is a divalent group having an aromatic ring,
R¹¹ is a group represented by the following Formula (11),

   (*-)₂Ar¹(-L¹-R^{N+})ₙ(A^{c-})_{n/c} ··· (11)
Ar¹ is a 2+n valent group having an aromatic ring,
L¹ is a single bond or divalent hydrocarbon group,
R^{N+} is a group having a ring structure containing N⁺,
A^{c-} is a c valent counter anion,
n is an integer of 1 or more,
c is 1 or 2, and
* represents a bonding hand with Ar².

The polymer of the present disclosure (hereinafter also referred to as polymer (A)) is a polymer having at least one structural unit (2) described above, and may have further other structural units to the extent that they exert the effects of the present disclosure. The polymer is a linear polymer having the above structural unit (2) in which R¹¹ and Ar² are alternately arranged, and optionally other structural units. In addition, it is preferable that the aromatic ring possessed by R¹¹ and the aromatic ring possessed by Ar² are bonded by a single bond to form the main chain. Due to this configuration, the main chain skeleton is free of ether oxygen (-O-), sulfonyl (-S(=O)₂-), carbonyl (-C(=O)-) bonds, thereby exhibiting excellent chemical durability, particularly alkali durability.

R¹¹ is a group represented by the above Formula (11), and each sign in the Formula (11) is the same as the above Formula (1) and each preferred aspect is also the same.

Ar² is a divalent group having an aromatic ring. It is preferable that Ar² does not have an ionic functional group. Ar² includes, in terms of chemical durability, a group in which an aromatic ring without an ionic functional group or two or more aromatic rings without an ionic functional group is linked via a single bond or a spiro atom. Ar² has an aromatic ring similar to those of Ar¹. In addition, the aromatic ring of Ar² may have substituent other than those having ionic functionality. Examples of the substituent include a halogen atom, an alkyl group having 1 to 20 carbon atoms, which may have a substituent, a phenyl group, which may have a substituent, and the like. Halogen atoms include F, Cl, Br, I, but F is desirable from a synthetic point of view. Specific examples of the above alkyl group include alkyl group such as a methyl group, an ethyl group, a propyl group, an n-butyl group, a tert-butyl group, a pentyl group, a hexyl group, and an octyl group, etc. and may have a phenyl group, etc. as a substituent. In addition, the substituent that the phenyl group may have include an alkyl group having 1 to 6 carbon atoms, and the like.

In terms of the excellent mechanical strength, chemical durability, and membrane formability of the polymer (A), the Ar² is preferably a divalent aromatic group that may have substituent other than those having ionic functionality, among which the group represented by any of the following Formulas (b1) to (b10) is preferred.

It should be noted that the plurality of Ar² in the polymer may be the same or different from each other. 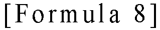 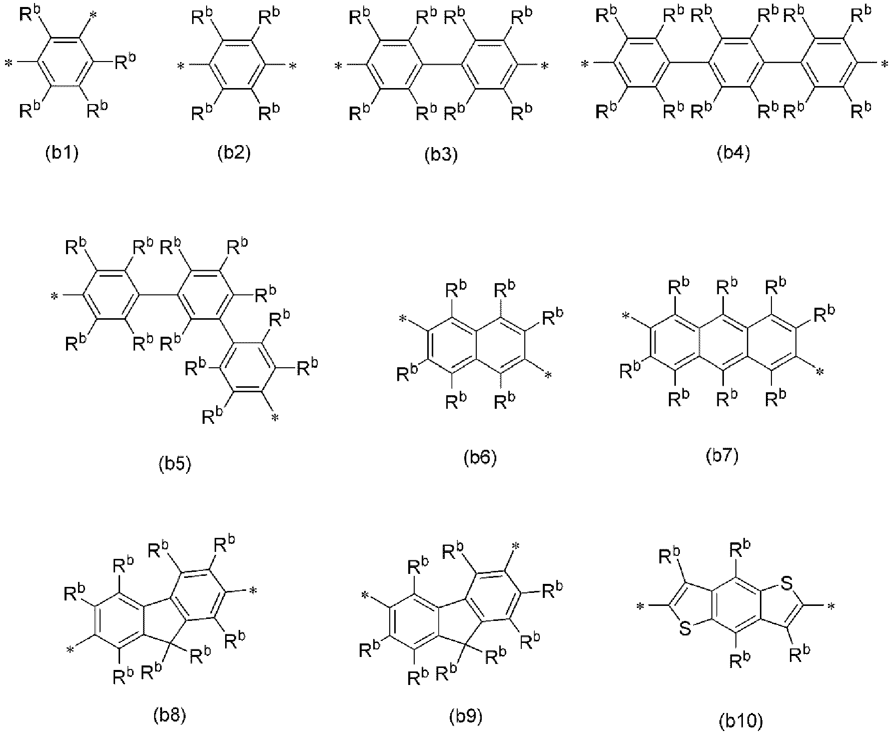 Where, R^{b} is each independently a group that does not have a hydrogen atom or an ionic functional group, and * represents a bonding hand that binds to R¹¹.

Furthermore, from the point of view that it is easy to produce a polymer with a high molecular weight, it is preferable to have a fluorine atom in the α-position relative to the bonding hand with R¹¹. The use of the polymer of high molecular weight makes it possible to form a membrane with better mechanical strength.

As for Ar² having a fluorine atom in the α-position, a group represented by any of the following Formulas (c1) to (c9) is particularly preferable. 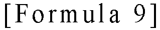 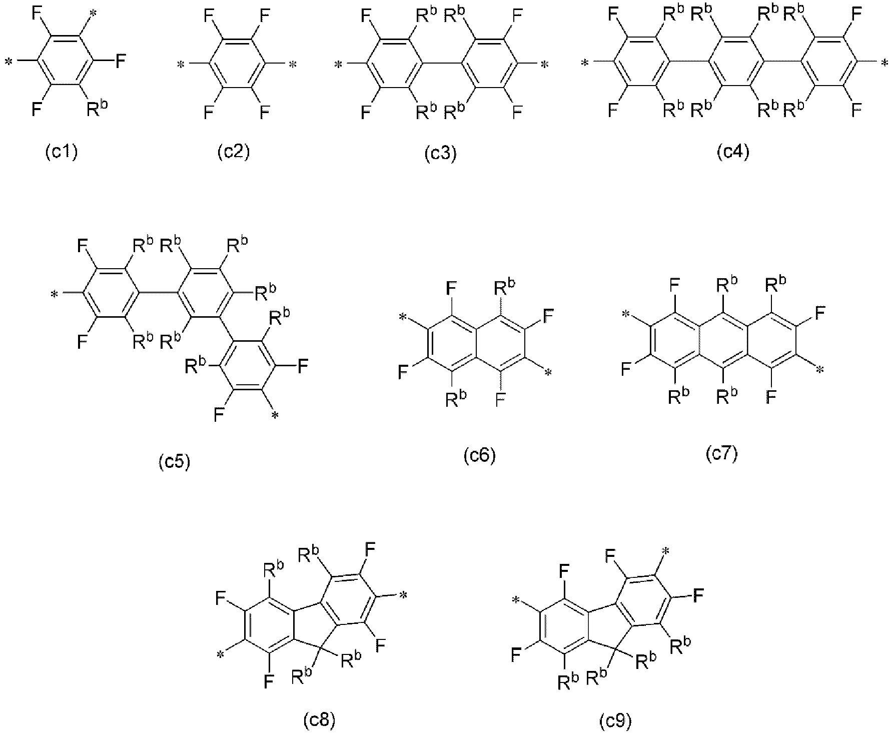 Where, R^{b} is each independently a group that does not have a hydrogen atom or an ionic functional group, and * represents a bonding hand that binds to R¹¹.

### [Other structural units]

The polymer of the present disclosure may have other configurations to the extent that they exert the effect of the present disclosure. Other structural units include, for example, structural unit represented by the following Formula (3), and the like. 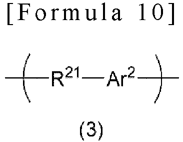 Where, Ar² is a divalent group having an aromatic ring,
R²¹ is a divalent group having an aromatic ring.

Ar² in structural unit (3) is similar to Ar² in the structural unit (2). In addition, R²¹ includes a group that does not have a ring structure containing N⁺ in R¹¹ of structural unit (2). R²¹ may have the structure represented by the following Formula (21), for example.

(*-)₂Ar¹(-R²²)ₘ ... (21)

Where, Ar¹ is a 2+m valent group having an aromatic ring,
R²² is a group having no ionic functional group,
m is an integer of 0 or more.

Ar¹ in Formula (21) is the same as Ar¹ in Formula (11), except that n is read as m. R²² includes, for example, an alkyl group having 1 to 20 carbon atoms that may have a substituent other than an ionic functional group, a phenyl group that may have a substituent, and the like.

The weight average molecular weight of the polymer (A) can be appropriately adjusted, for example, it can be in the range of 10,000 to 1,000,000, preferably 50,000 or more, more preferably 100,000 or more, in view of membrane formability, membrane strength, and the like. The weight average molecular weight is a polystyrene equivalent value measured by GPC (gel permeation chromatography).

The proportion of the structural unit (2) in the polymer (A) is preferably 50 mass% or more relative to the total mass of the polymer.

### <Method for producing polymer (A)>

Although the method for producing the polymer (A) is not particularly limited, it is preferable, for example, to include at least a step of reacting a compound represented by the above Formula (1) with a compound represented by H-Ar²-H (hereinafter also referred to as compound (4)). In addition, in the compound represented by the Formula (1), substitutions of -L¹-R^{N+} with -L¹-X² (X² is a halogen atom) may be used, and X² may be substituted with R^{N+} after polymer synthesis.

Furthermore, by using Ar² of compound (4) having a fluorine atom in the α-position of H (e.g., the following Formulas (d1) to (d9)), it is possible to synthesize relatively easily an ion conductive polymer of high molecular weight (for example, weight-average molecular weight is 100,000 or more) having excellent reactivity between X¹ of compound (1) and a hydrogen atom of H-Ar²-H. 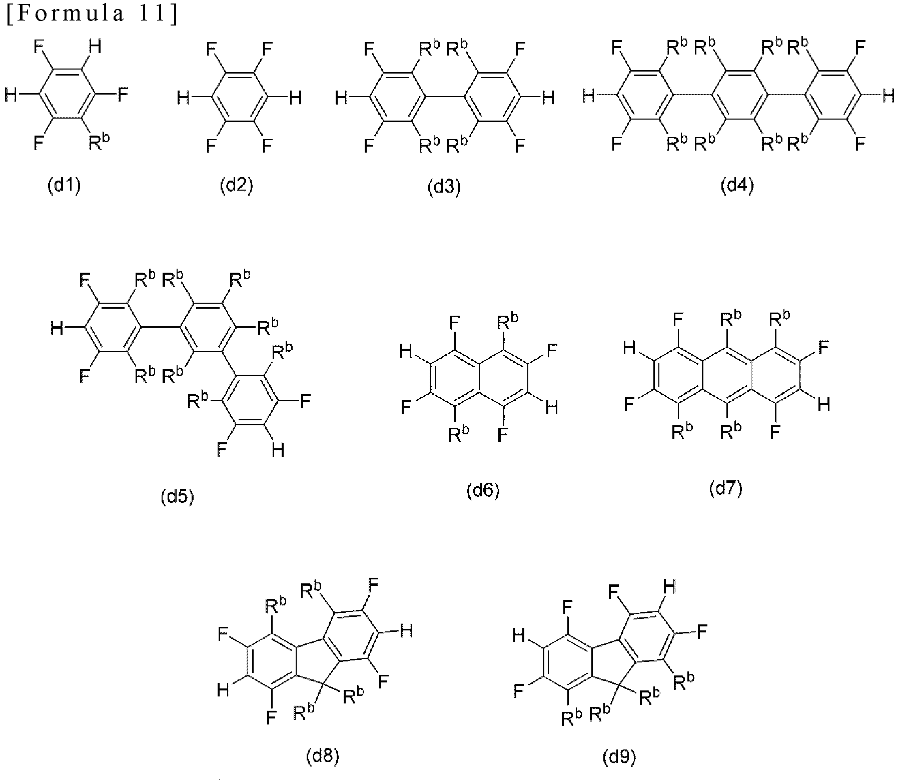 Where, R^{b} is each independently a hydrogen atom or a substituent that having no ionic functional group.

The polymerization of compound (1) with compound (4) can be performed by, for example, reacting compound (1) with compound (4) in a solvent in the presence of a Pd complex, a ligand, a carboxylic acid (RCO₂H) and a base. Various Pd complexes, ligands, carboxylic acids, bases, and solvents can be used in the reaction step. Among them, the use of each of Pd₂(dba)₃-CHCl₃, P(o-C₆H₄-OMe)₃, pivalic acid (PivOH), Cs₂CO₃, and dryTHF is particularly preferred. Here, dba means dibenzylideneacetone. Here, the reaction time and reaction temperature in the reaction step can also be appropriately set, and may be, for example, 1 to 48 hours and 80 to 140°C, respectively.

Then, if X² was substituted in the position of R^{N+} of compound (1), polymer (A) is obtained by introducing the desired R^{N+} after polymerization. The method for introducing R^{N+} may be referred to in the examples described below, as well as in the methods disclosed in Japanese Unexamined Patent Application Publication No. 2020-169287 and Japanese Unexamined Patent Application Publication No. 2021-042351.

### 3. Electrolyte membrane

The characteristic feature of the electrolyte membrane according to the present disclosure is the inclusion of polymer (A). The electrolyte membrane using polymer (A) is excellent in chemical durability, ionic conductivity, and membrane mechanical strength, and can be suitably used as an electrolyte membrane for a fuel cell and electrolysis apparatus. In addition, the polymer having an ion exchange group can be easily dissolved in a solvent (e.g., alcohol or a mixed solvent of alcohol and water) normally used in the production of the membrane electrode assembly (MEA) of a fuel cell, and also has excellent gas permeability. Therefore, the polymer can also be used as an electrolyte ionomer in these batteries and electrolytic devices.

The polymer (A) has a high ionic functional group density (IEC) and exhibits very high ionic conductivity. The IEC of the polymer is preferably 0.5 meq•g⁻¹ or more and 4.0 meq•g⁻¹ or less. The ionic conductivity of the polymer preferably has an ionic conductivity of 50 mS/cm or higher at 80°C and saturated humidity. The polymer has good swelling resistance, whereas general polymers with high ionic conductivity have a high water content and often swells remarkably in a water-containing state.

A general membrane-forming method can be applied to the production of electrolyte membranes. For example, an electrolyte membrane can be obtained by dissolving the polymer in a solvent capable of dissolving it (e.g., dimethylsulfoxide, alcohol, or an alcohol aqueous solution) to prepare a polymer solution, making the solution into a coating film using a known coating means, and drying it.

### 4. Fuel cell

The characteristic feature of the cell according to the present disclosure is to have the electrolyte membrane. The electrolyte membrane can be suitably used for both solid alkaline fuel cells and solid polymer electrolyte fuel cells.

In a case where the electrolyte membrane is applied to a solid alkaline fuel cell, an anion conductive electrolyte membrane is used as the electrolyte membrane. The configuration of the solid alkaline fuel cell may be a conventionally known configuration.

For example, a membrane electrode assembly is formed by arranging a cathode on one side of the electrolyte membrane and an anode on the other side thereof; then, oxygen is supplied to the cathode and fuel is supplied to the anode; subsequently, OH⁻ generated at the cathode is transferred to the anode through the electrolyte membrane to produce water, and electricity is thus generated.

The fuel can be appropriately selected from conventionally known fuels, and examples thereof include, but are not limited to, hydrogen, methanol, ethanol, ethylene glycol, formate, hydrazine, sodium borohydride, and ammonia.

The followings are representative examples of the reactions at the respective electrodes when hydrogen, methanol, or formate is used as the fuel.
- Fuel cell with hydrogen
   Anode:

      2OH⁻ + H₂ → 2H₂O
   Cathode:

      O₂ + 2H₂O + 4e⁻ → 4OH⁻
- Fuel cell with methanol
   Anode:

      6OH⁻ + CH₃OH → CO₂ + 5H₂O
   Cathode:

      O₂ + 2H₂O + 4e⁻ → 4OH⁻
- Fuel cell with formate
   Anode:

      HCOO⁻ + 3OH⁻ → 2H₂O + CO₃²⁻ + 2e⁻
   Cathode:

      O₂ + 2H₂O + 4e⁻ → 4OH⁻

In a case where the electrolyte membrane is applied to a solid polymer electrolyte fuel cell, a proton conductive electrolyte membrane is used as the electrolyte membrane. The configuration of the solid polymer electrolyte fuel cell may be a conventionally known configuration.

For example, a membrane electrode assembly is formed by arranging a cathode on one side of the electrolyte membrane and an anode on the other side thereof; then, oxygen is supplied to the cathode and fuel is supplied to the anode; subsequently, protons generated at the anode is transferred to the anode through the electrolyte membrane to produce water, and electricity is thus generated.

The fuel can be appropriately selected from conventionally known fuels, and specific examples thereof include those exemplified for the above solid alkaline fuel cell.

The followings are representative examples of the reactions at the respective electrodes when hydrogen is used as a fuel.
Anode:

   H₂ → 2H⁺ + 2e⁻
Cathode:

   O₂ + 4H⁺ + 4e⁻ → 2H₂O

### 5. Electrolysis apparatus

The electrolyte membrane of the present disclosure can be suitably used for water electrolysis, other electrolysis techniques (electrolysis method), and electrolysis apparatus using these electrolysis methods. The electrolysis apparatus may have, for example, the electrolyte membrane of the present disclosure, an anode and a cathode in an electrolytic cell, and the target object can be obtained through electrolysis (oxidation-reduction reaction) of a material of interest via the electrolyte membrane of the present disclosure.

In a case where the electrolyte membrane is applied to a water electrolysis apparatus, a proton-conducting or anion-conducting electrolyte membrane is used as the electrolyte membrane. For example, an anode is arranged on one side of a proton-conducting electrolyte membrane and a cathode is arranged on the other side thereof, and protons generated at the anode are transferred to the cathode through the electrolyte membrane and combined with electrons at the cathode to obtain hydrogen. The formulas of reactions at the respective electrodes are as follows.
Anode:

   2H₂O → O₂ + 4H⁺ + 4e⁻
Cathode:

   2H⁺ + 2e⁻ → H₂

Examples of other electrolysis techniques include an electrolysis technique in which carbon dioxide is electrolyzed to generate formic acid. For example, protons generated at the anode can be transferred to the cathode through the electrolyte membrane and reacted with carbon dioxide supplied to the cathode to obtain formic acid. The formulas of reactions at the respective electrodes are as follows.
Anode:

   2H₂O → O₂ + 4H⁺ + 4e⁻
Cathode:

   CO₂ + 2H⁺ + 2e⁻ → HCOOH

### Examples

Hereinafter, the present invention will now be described more specifically by way of examples and comparative examples. However, these descriptions for examples and comparative examples do not limit the present invention.

### [Synthesis Example 1: Synthesis of compound i]

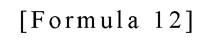

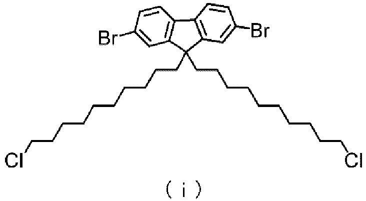

In a two-neck flask, sodium hydroxide (150 g) aqueous solution (300 mL) was added with n-tetrabutylammonium chloride (556 mg) and the mixture was stirred under a nitrogen. 2,7-dibromofluorene (4.86 g, 15 mmol) was dissolved in 1,10-dichlorohexane (31.7 g, 150 mmol) under heating and this solution was added to the two-neck flask using a syringe.

After reacting for 120 minutes under 95°C nitrogen, the solution was allowed to cool to room temperature. The organic phase was extracted with dichloromethane (300 mL) and washed with 1M hydrochloric acid (50 mL) and water (200 mL × 2). Dichloromethane was evaporated in an evaporator and unreacted 1,10-dichlorodecane was removed at 90°C under reduced pressure. The obtained residue was applied to a silica gel column (elution solvent: hexane), yielding the target compound i (2,7-dibromo, 9,9'-(10-chlorodecane), 8.14 g, 12.1 mmol).

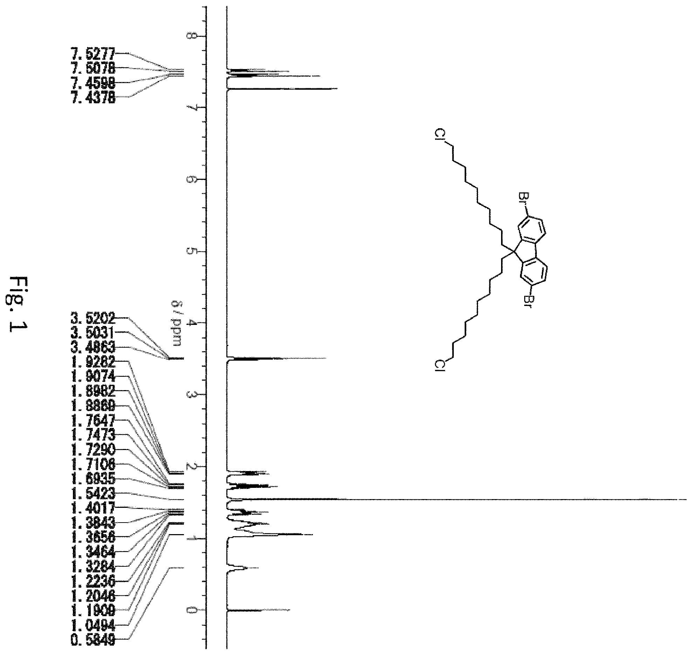

### (¹H-NMR spectrum of compound i (see Fig. 1))

¹H-NMR (400 MHz, CDCl₃): 7.52 (2H, m), δ 7.45 (4H, m), δ 3.50 (4H, t), δ 1.91 (4H, t), δ 1.73 (4H, m), δ 1.37 (4H, m), δ 1.20 (8H, m), δ 1.05 (12H, m), δ 0.58 (4H, m).

### [Synthesis Example 2: Synthesis of compound ii]

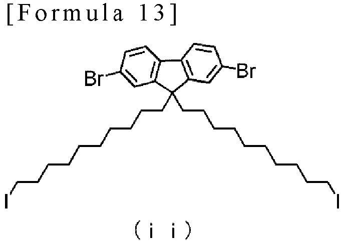

2,7-Dibromo, 9,9'-(10-chlorodecane) (6.73 g, 10 mmol) was dissolved in acetone (100 mL), and sodium iodide (7.5 g, 50 mmol) was added and the mixture was stirred at 70°C for 12 hours. The solvent was removed with an evaporator, chloroform was added to the residue, filtered, and then chloroform was removed with an evaporator. The residue was applied to a silica gel short column (elution solvent: hexane) and hexane was removed with an evaporator. To the obtained residue, acetone (100 mL) and sodium iodide (7.5 g, 50 mmol) were added again and the mixture was stirred at 70°C for 12 hours. Purification operations similar to those described above were performed and applied to a silica gel short column (elution solvent: hexane), yielding the target compound ii (2,7-dibromo, 9,9'-(10-chlorodecane), 6.51 g, 7.6 mmol).

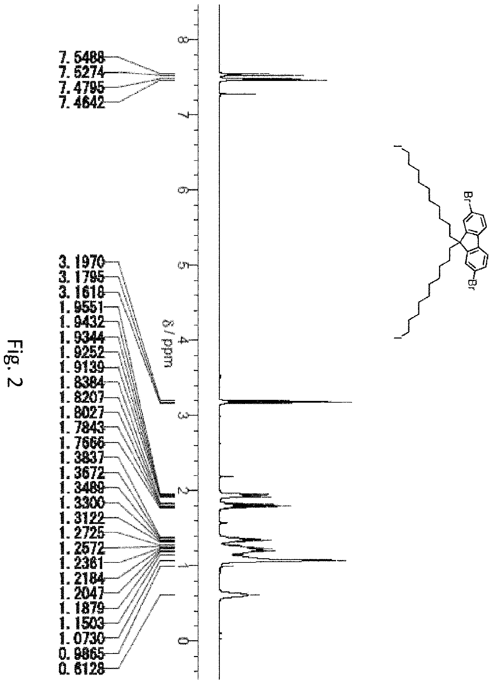

### (¹H-NMR spectrum of compound ii (see Fig. 2))

¹H-NMR (400 MHz, CDCl₃): 7.53 (2H, m), δ 7.46 (4H, m), δ 3.18 (4H, t), δ 1.93 (4H, t), δ 1.80 (4H, m), δ 1.34 (4H, m), δ 1.24 (8H, m), δ 1.07 (12H, m), δ 0.61 (4H, m).

### [Example 1: Synthesis of Qin]

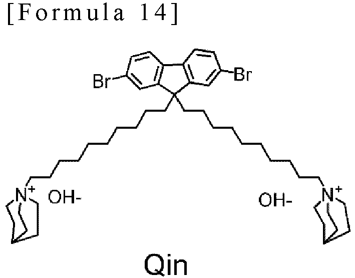

To 2,7-dibromo, 9,9'-(10-chlorodecane) (202 mg, 0.3 mmol) was added quinuclidine (222 mg, 2 mmol), 1,4-dioxane (10 ml) and reacted at 70°C for 3 hours. Methanol (10 ml) was then added to the mixture and allowed to react at 70°C for 18 hours. The solvent was removed with an evaporator and dried in vacuo to give the target compound (Qin, 260 mg).

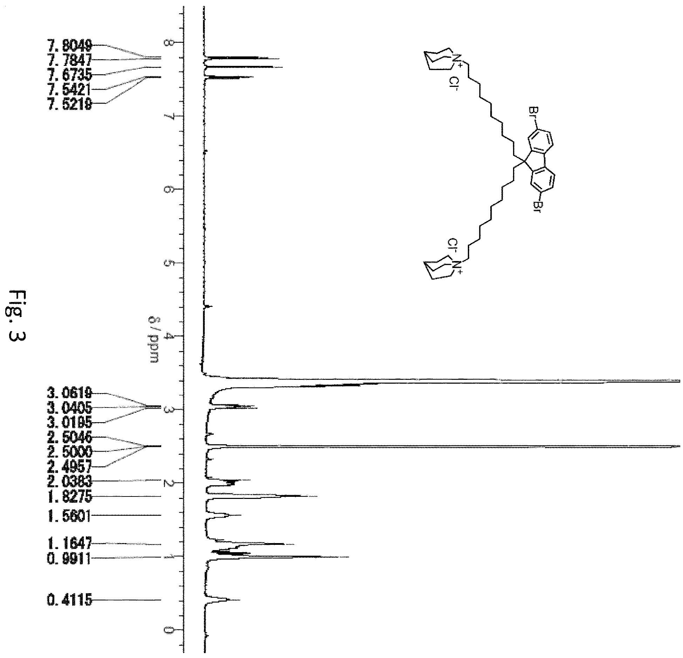

### (¹H-NMR spectrum of Qin (see Fig. 3))

¹H-NMR (400 MHz, DMSO-d6): 7.79 (2H, d), δ 7.67 (2H, s), δ 7.53 (2H, d), δ 3.24 (12H, t), δ 3.03 (4H, s), δ 2.03 (6H, m), δ 1.82 (12H, m), δ 1.56 (4H, m), δ 1.18 (12H, m), δ 0.99 (12H, m), δ 0.41 (4H, m).

### [Comparative Example 1: Synthesis of TMA]

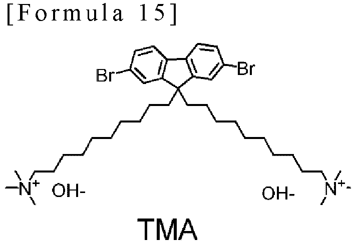

To 2,7-dibromo, 9,9'-(10-iododecane) (257 mg, 0.3 mmol) was added 25% trimethylamine methanol solution (1 ml, 3.2 mmol), 1,4-dioxane (15 ml) and allowed to react at 70°C for 3 hours. Methanol (10 ml) was then added to the mixture and allowed to react at 70°C for 12 hours. The solvent was removed with an evaporator and dried in vacuo to give the target compound (TMA, 278 mg).

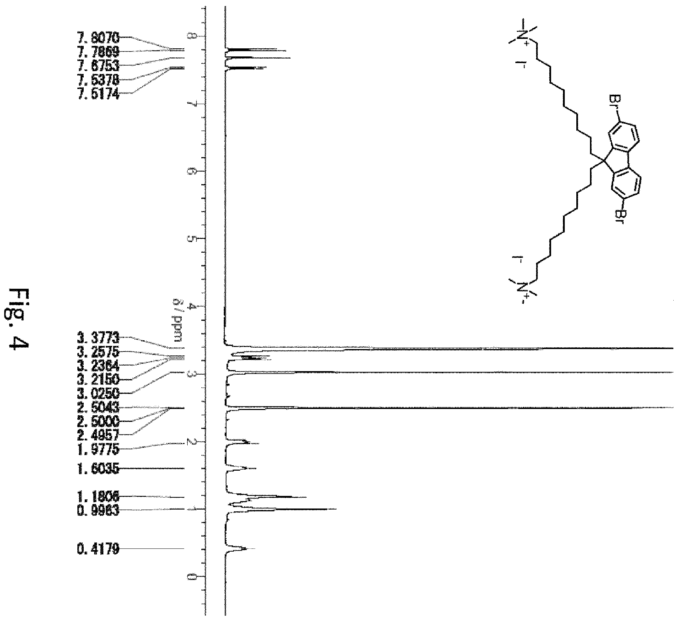

### (¹H-NMR spectrum of TMA (see Fig. 4))

¹H-NMR (400 MHz, DMSO-d6): 7.79 (2H, d), δ 7.68 (2H, s), δ 7.52 (2H, d), δ 3.24 (4H, t), δ 3.03 (18H, s), δ 1.98 (4H, t), δ 1.60 (4H, m), δ 1.18 (12H, m), δ 1.00 (12H, m), δ 0.42 (4H, m).

### [Comparative Example 2: Synthesis of TEA]

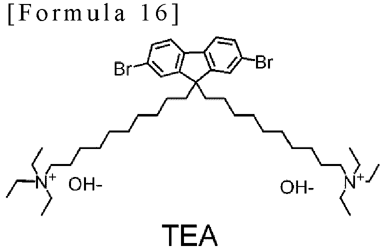

In Comparative Example 1 above, instead of trimethylamine, triethylamine (300 mg, 3 mmol) was used to perform a similar reaction and purification procedure to give the target compound (TEA 302 mg).

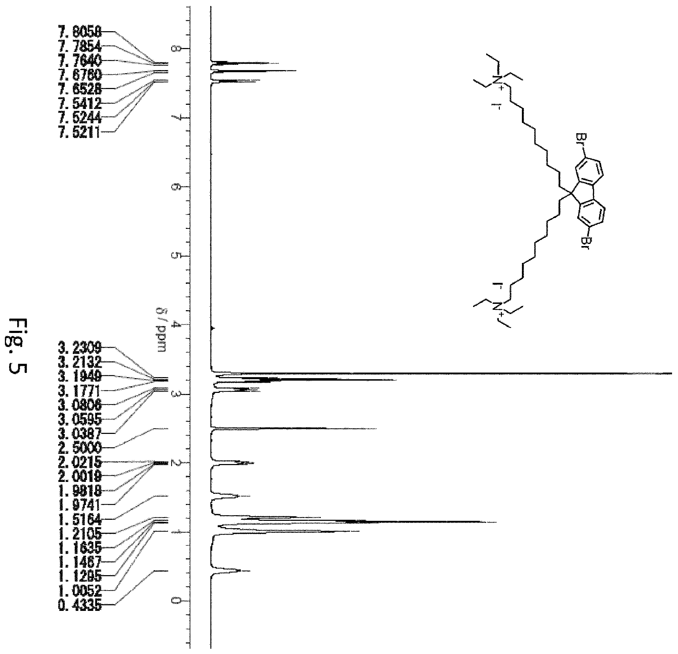

### (¹H-NMR spectrum of TEA (see Fig. 5))

¹H-NMR (400 MHz, DMSO-d6): 7.79 (2H, d), δ 7.68 (2H, s), δ 7.52 (2H, d), δ 3.21 (4H, t), δ 3.08 (12H, q), δ 2.00 (4H, t), δ 1.52 (8H, m), δ 1.15 (26H, m), δ 1.00 (12H, m), δ 0.43 (4H, m).

### [Comparative Example 3: Synthesis of DiPrM]

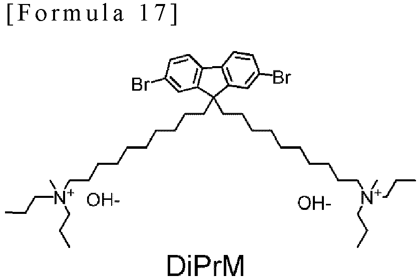

In Comparative Example 2 above, instead of triethylamine, dimethylpropylamine (230 mg, 2 mmol) was used to perform a similar reaction. After the reaction, the solvent was removed with an evaporator, then hexane was added and washed, and then dried in vacuo to give the target compound (DiPrM 287 mg).

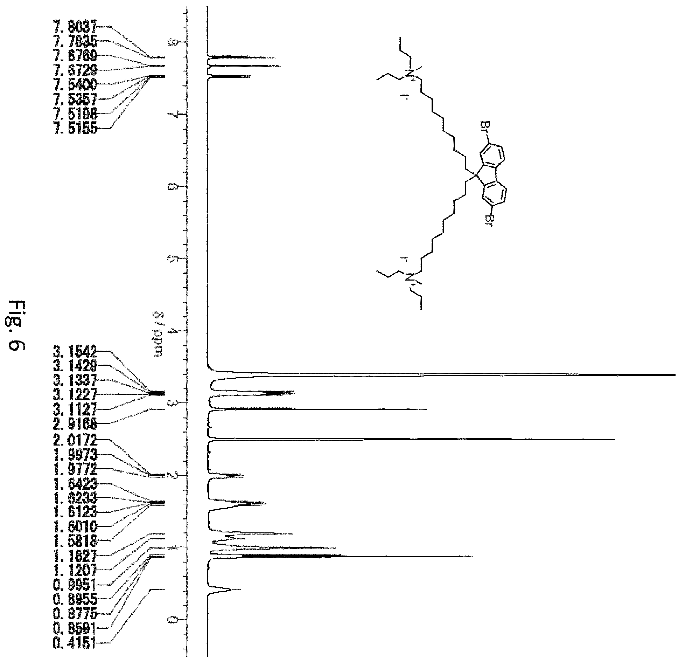

### (¹H-NMR spectrum of DiPrM (see Fig. 6))

¹H-NMR (400 MHz, DMSO-d6): 7.79 (2H, d), δ 7.68 (2H, s), δ 7.52 (2H, d), δ 3.13 (12H, m), δ 2.92 (6H, s), δ 2.00 (4H, t), δ 1.61 (12H, m), δ 1.18 (8H, m), δ 1.12 (4H, m), δ 1.00 (12H, m), δ 0.88 (12H, m), δ 0.42 (4H, m).

### [Example 2: Synthesis of Pyr]

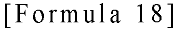

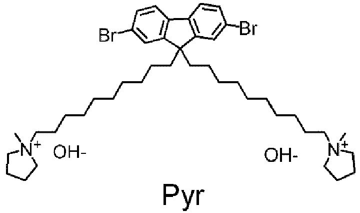

To 2,7-dibromo, 9,9'-(10-iododecane) (257 mg, 0.3 mmol) was added 1-methylpyrrolidine (170 mg, 2 mmol), 1,4-dioxane (15 ml) and allowed to react at 70°C for 3 hours. Methanol (10 ml) was then added to the mixture and allowed to react at 70°C for 12 hours.
The solvent was removed with an evaporator, then hexane was added and washed, and then dried in vacuo to give the target compound (Pyr 277 mg).

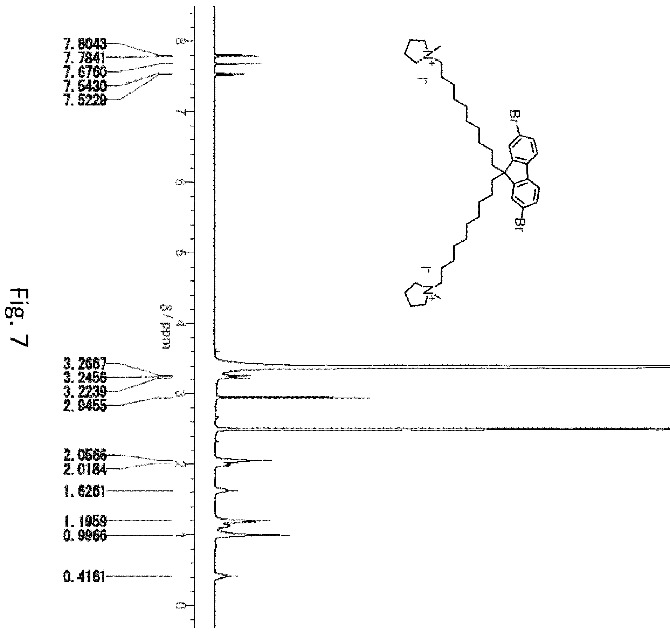

### (¹H-NMR spectrum of Pyr (see Fig. 7))

¹H-NMR (400 MHz, DMSO-d6): 7.79 (2H, d), δ 7.68 (2H, s), δ 7.53 (2H, d), δ 3.43 (8H, m), δ 3.24 (4H, t), δ 2.94 (6H, s), δ 2.06 (8H, m), δ 2.00 (4H, t), δ 1.62 (4H, m), δ 1.20 (8H, m), δ 1.13 (4H, m), δ 1.00 (12H, m), δ 0.41 (4H, m).

### [Example 3: Synthesis of Pep]

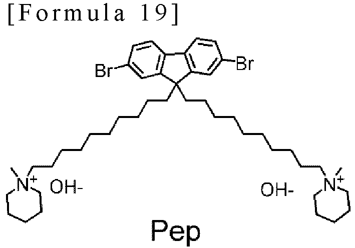

In Example 2, instead of 1-methylpyrrolidine, 1-methylpiperidine (198 mg, 2 mmol) was used to perform a similar reaction. After the reaction, the solvent was removed with an evaporator, then hexane was added and washed, and then dried in vacuo to give the target compound (Pep 281 mg).

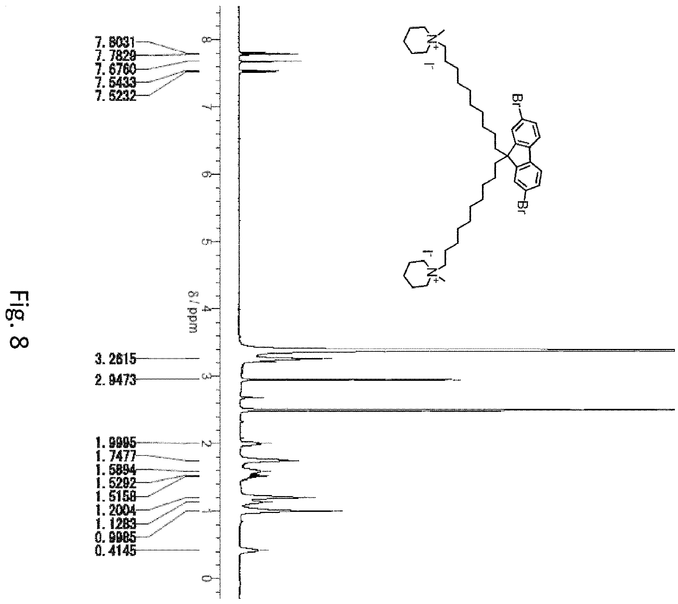

### (¹H-NMR spectrum of Pep (see Fig. 8))

¹H-NMR (400 MHz, DMSO-d6) 7.79 (2H, d), δ 7.68 (2H, s), δ 7.52 (2H, d), δ 3.26 (12H, m), δ 2.94 (6H, s), δ 2.00 (4H, t), δ 1.74 (8H, m), δ 1.59 (4H, m), δ 1.53 (4H, m), δ 1.20 (8H, m), δ 1.13 (4H, m), δ 1.00 (12H, m), δ 0.41 (4H, m).

### [Comparative Example 4: Synthesis of DMOc]

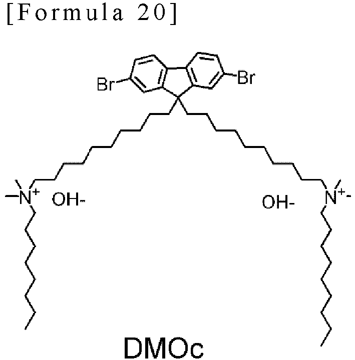

In Comparative Example 1, instead of trimethylamine, dimethyloctylamine (314 mg, 2 mmol) was used to perform a similar reaction. After the reaction, solvent was removed with an evaporator, then hexane was added and washed, and then dried in vacuo to give the target compound (DMOc 309 mg).

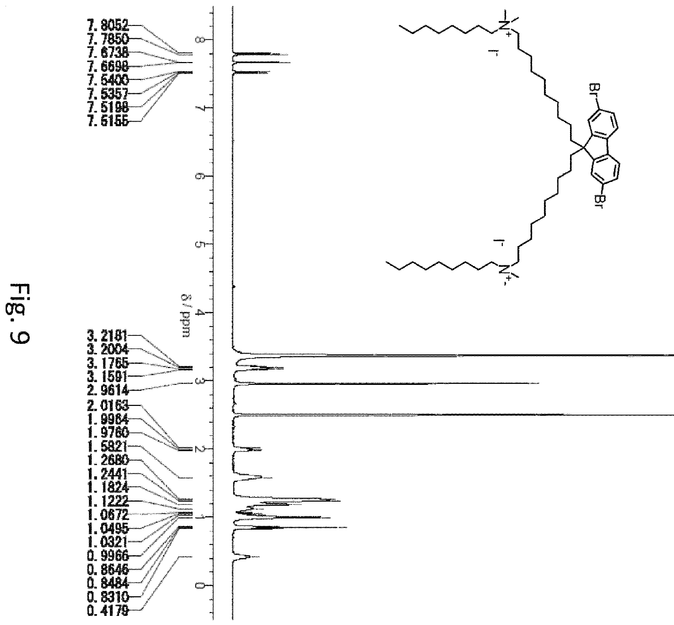

### (¹H-NMR spectrum of DMOc (see Fig. 9))

¹H-NMR (400 MHz, DMSO-d6): 7.79 (2H, d), δ 7.68 (2H, s), δ 7.52 (2H, d), δ 3.20 (8H, m), δ 2.96 (12H, s), δ 2.00 (4H, t), δ 1.58 (8H, m), δ 1.25 (20H, m), δ 1.18 (8H, m), δ 1.12 (4H, m), δ 1.00 (12H, m), δ 0.85 (6H, t), δ 0.42 (4H, m).

### [Synthesis Example 3: Synthesis of Polymer I]

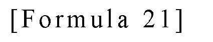

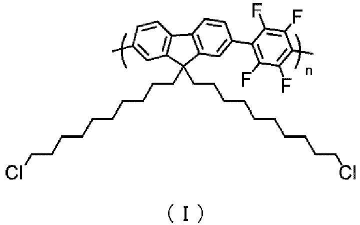

2,7-dibromo, 9,9'-(10-chlorodecane) (1346 mg, 2 mmol), cesium carbonate (1.96 g, 6 mmol), pivalic acid (204 mg, 2 mmol), tris(2-methoxyphenyl) phosphine (14 mg), and Pd₂(dba)₃·CHCl₃ complex (10.4 mg) were added to two-neck flask, then dehydrated tetrahydrofuran (3 mL) was added and the mixture was stirred under nitrogen. Tetrafluorophenylene (309 mg, 2.06 mmol) was dissolved in tetrahydrofuran (1 mL) and added to the two-neck flask using a syringe to minimize the entry of air. After reacting under nitrogen at room temperature for 45 minutes, the reaction was performed at 80°C for 24 hours.

To the obtained solid, 1M hydrochloric acid and chloroform were added, and the mixture was stirred at 40°C for about 1 hour, and then an organic phase was extracted with a separatory funnel. The organic phase was washed with water and then dried in an evaporator. The obtained residue was dissolved in chloroform and reprecipitated in methanol. The precipitation was filtered and then washed with hexane. The obtained solid was dried in vacuo to give the target polymer I (1271 mg).

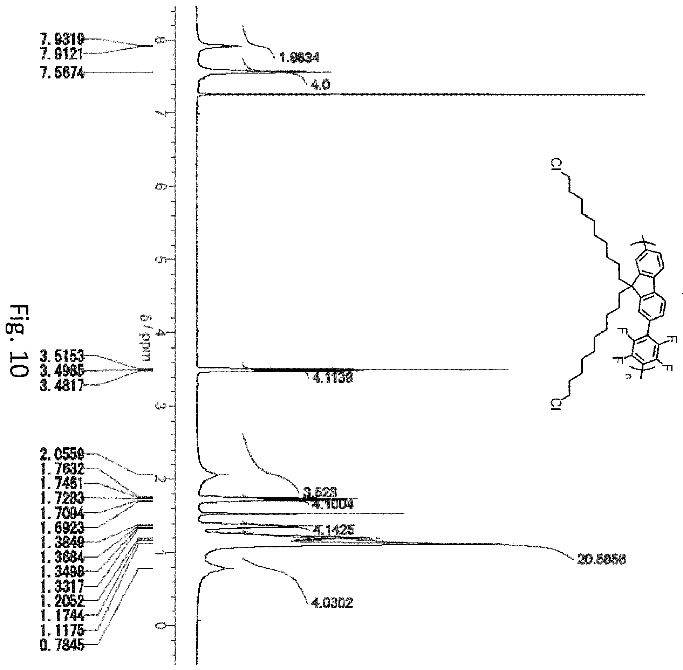

### (¹H-NMR spectrum of polymer I (see Fig. 10))

¹H-NMR (CDCl3, 400 MHz): δ 7.92 (2H, m), δ 7.57 (4H, m), δ 3.50 (4H, t), δ 2.06 (4H, t), δ 1.73 (4H, m), δ 1.37 (4H, m), δ 1.17 (20H, m), δ 0.78 (4H, m)

### [Synthesis Example 4: Synthesis of polymer II]

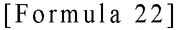

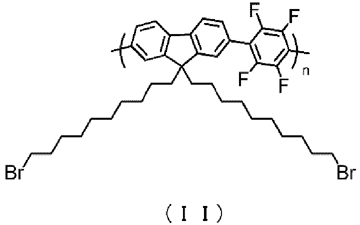

The polymer I (1.0 g) was dissolved in chlorobenzene (50 ml), and tetrabutylammonium bromide (322.3 mg, 1 mmol) and 1,2-dibromoethane (3.76 g, 20 mmol) were added, and the mixture was stirred at 130°C for 12 hours. The solvent was concentrated in an evaporator and then the polymer was reprecipitated in methanol. The obtained solid was filtered and dried in vacuo to give the target polymer II (1010 mg).

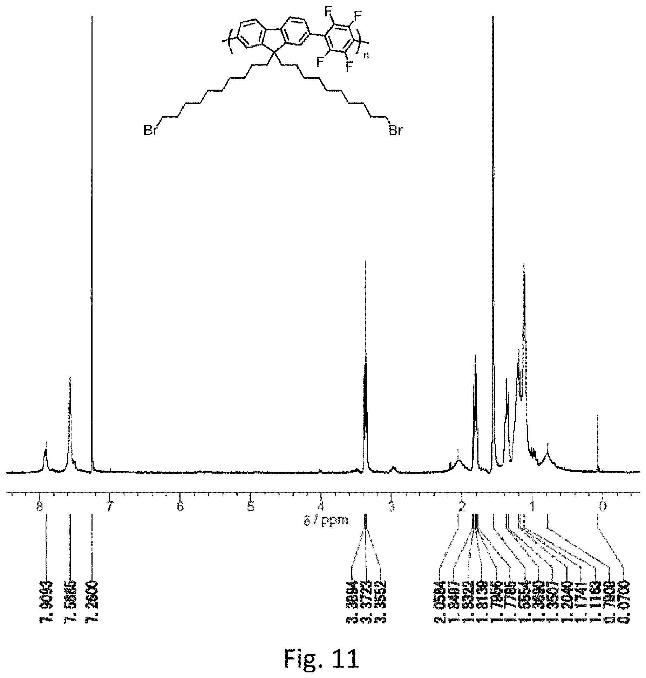

### (¹H-NMR spectrum of polymer II (see Fig. 11))

¹H-NMR (CDCl3, 400 MHz): δ 7.91 (2H, m), δ 7.57 (4H, m), δ 3.37 (4H, t), δ 2.06 (4H, t), δ 1.81 (4H, m), δ 1.36 (4H, m), δ 1.17 (20H, m), δ 0.79 (4H, m)

### [Example 4: Synthesis of polymer III]

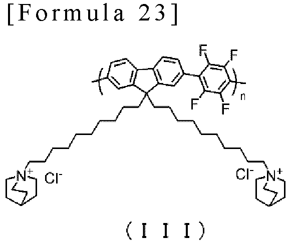

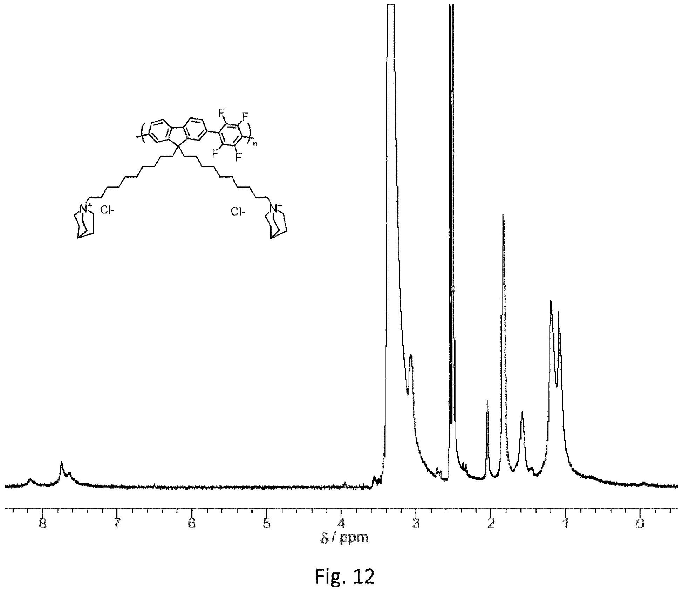

Polymer I (200 mg) was dissolved in chlorobenzene (15 ml), and quinuclidine (222 mg, 2 mmol) was added, and the mixture was stirred at 105°C for 3 hours. Then dimethylsulfoxide (15 ml) was added and the mixture was stirred for 12 hours. After removing the solvent in an evaporator, the polymer was washed with water and hexane and dried in vacuo to give the target polymer III (205 mg). ¹H-NMR spectrum of polymer III is shown in Fig. 12.

### [Example 5: Synthesis of polymer IV]

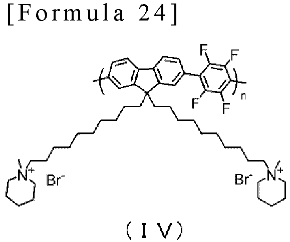

Polymer II (200 mg) was dissolved in chlorobenzene (15 ml), and 1-methylpiperidine (298 mg, 3 mmol) was added, and the mixture was stirred at 80°C for 2 hours. Then dimethylsulfoxide (15 ml) was added and the mixture was stirred for 6 hours. After removing chlorobenzene in an evaporator, dimethylsulfoxide (15 ml) and 1-methylpiperidine (99 mg, 1 mmol) were added and the mixture was stirred for 6 hours. After removing the solvent in an evaporator, the polymer was washed with water and hexane and dried in vacuo to give the target polymer IV (198 mg).

### [Comparative Example 5: Synthesis of polymer V]

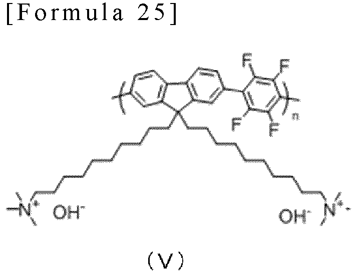

In Example 4, polymer V was obtained in the same way as in Example 4 except that trimethylamine was used instead of quinuclidine.

### [Evaluation]

### <Alkali durability test of compound>

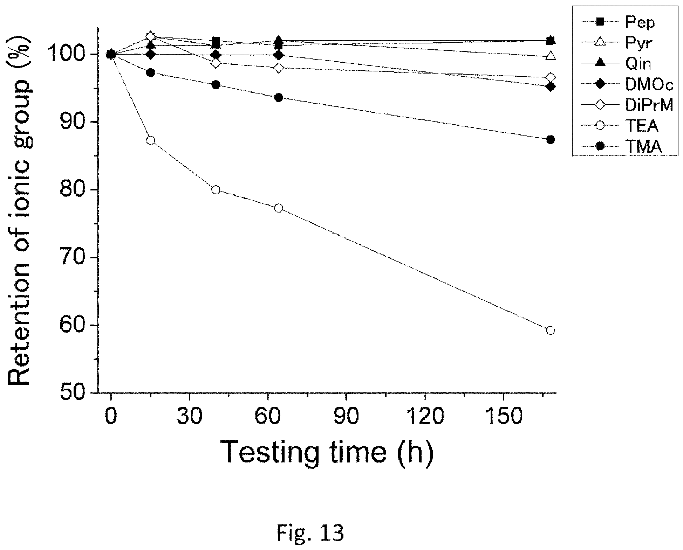

Alkaline durability tests were performed on the compounds of Examples 1 to 3 and Comparative Examples 1 to 4. Specifically, each compound (0.03 mmol) was added to 5 ml of 4M NaOH solution (water: 1 ml, methanol: 4 ml) and left in a thermostatic bath at 80°C. After a predetermined time had elapsed, a portion of the reaction solution was sampled and hydrochloric acid was added to neutralize. After the solvent was removed by vacuum drying, the obtained residue was extracted with dichloromethane and ethanol. After removing the solvent in an evaporator and the residue was dried in vacuo, the H-NMR spectrum of the obtained compound was measured and compared with the pre-test spectrum to evaluate the retention rate of ionic functional group in the model compound. The results are shown in Fig. 13.

### <Alkali durability test of electrolyte membrane>

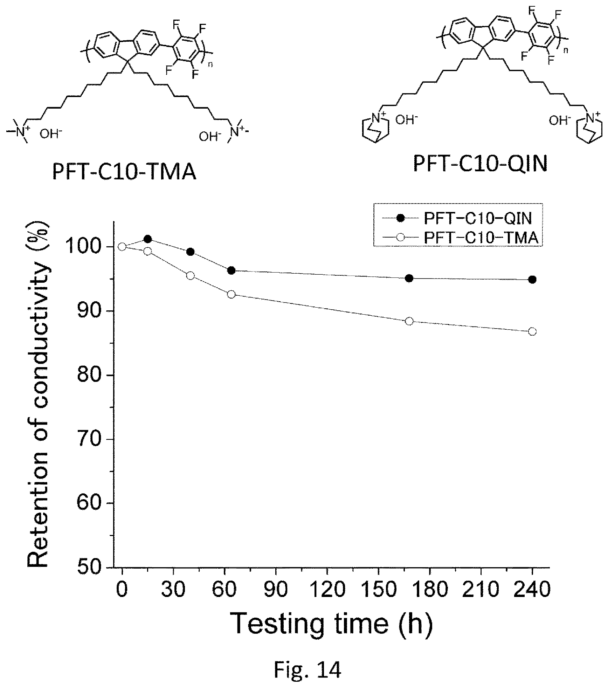

The polymers of Example 4 and Comparative Example 5 were dissolved in dimethylsulfoxide, respectively, and the solution was added dropwise onto the glass substrate at 100°C to produce an electrolyte membrane (membrane thickness: 25 µm). Each obtained electrolyte membrane was immersed in 1M NaOH solution for 24 hours, then immersed in pure water for 6 hours, and then the ionic conductivity of the electrolyte membrane was measured at 40°CRH95%. The electrolyte membrane was then immersed in 8M NaOH solution and left at 80°C. After a predetermined time had elapsed, the membrane was removed and immersed in pure water for 3 hours. After measuring the ionic conductivity of the membrane at 40°CRH95%, the membrane was immersed in 8M NaOH solution, and the same operation was repeated. The results are shown in Fig. 14.

As shown in Fig. 13, it was found that the compounds of Examples 1 to 3 having an ionic functional group with an N⁺ containing ring structure had a higher retention rate of the ionic functional groups and better alkali durability in an alkaline solution than the compounds of Comparative Examples 1 to 4. As also shown in Fig. 14, it was found that the electrolyte membrane using the polymer of Example 4 having the ionic functional groups with an N⁺ containing ring structure suppressed the decrease of ionic conductivity even under high temperature and strong alkaline condition.

Thus, the polymer of the present disclosure can be particularly suitably used for electrolyte membrane applications used in fuel cells and electrolytic apparatuses.

### •Synthesis of compound (1)

Furthermore, various compounds (1) were synthesized. All of the compounds in Examples 6 to 11 described below have excellent alkali durability.

### [Examples 6 to 8]

Sodium hydroxide (150 g) aqueous solution (300 mL) and n-tetrabutylammonium bromide (645 mg) were added to a two-neck flask, and the mixture was stirred under nitrogen. Fluorene (3.32 g, 20 mmol) was dissolved in 1,6-dibromohexane (48.7 g, 200 mmol) under heating and this solution was added to the two-neck flask using a syringe. After reacting for 90 minutes under 90°C nitrogen, the solution was allowed to cool to room temperature, and the organic phase was extracted with dichloromethane (300 mL) and washed with 1M hydrochloric acid (50 mL) and water (200 mL × 2). Dichloromethane was evaporated in an evaporator and unreacted 1,6-dibromohexane was removed at 90°C under reduced pressure. The obtained residue was applied to a silica gel column (elution solvent: hexane:chloroform = 9:1), yielding the following target compound A (6.45 g, 13.1 mmol).

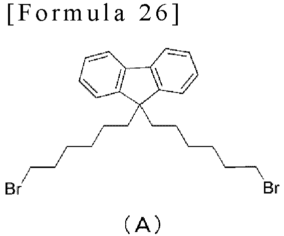

492 mg (1 mmol) of the above compound A was weighed out for each, and 5 ml of 1,4-dioxane was added. Then, each of the three amines (10 mmol) described below was added, and the reaction was performed at 80°C for 2 hours. 10 ml of ethanol was then added to the mixture and allowed to react for another 2 hours. After the solvent was removed under reduced pressure, the residue was washed with hexane to give the following three compounds.

The above amines used were 1-methylpiperidine (Pep), 1-methylpyrrolidine (Pyr), and quinuclidine (Qin).

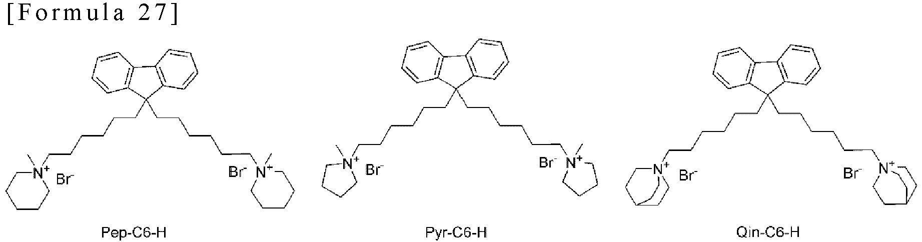

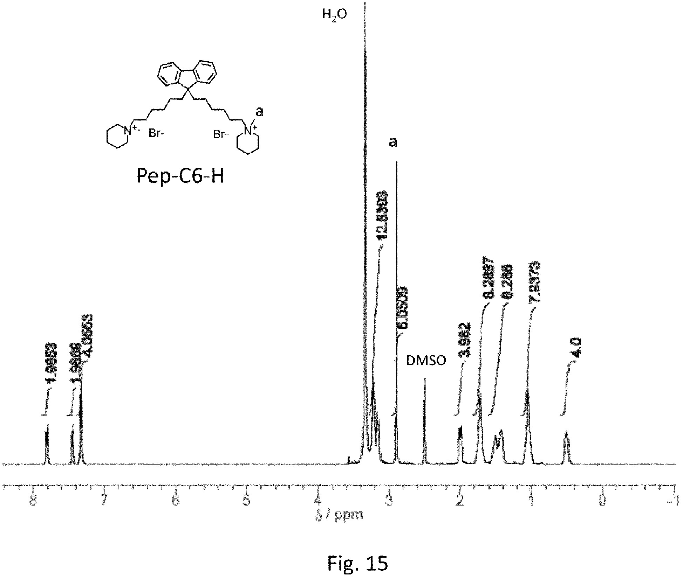

### (¹H-NMR spectrum of Pep-C6-H (see Fig. 15))

¹H-NMR (400 MHz, DMSO-d6): 7.81 (2H, m), δ 7.43 (2H, m), δ 7.32 (4H, m), δ 3.24 (8H, m), δ 3.15 (4H, t), δ 2.89 (6H, s), δ 2.00 (4H, m), δ 1.90 (8H, m)δ 1.72-1.40 (8H, m), δ 1.04 (8H, m), δ 0.50 (4H, m).

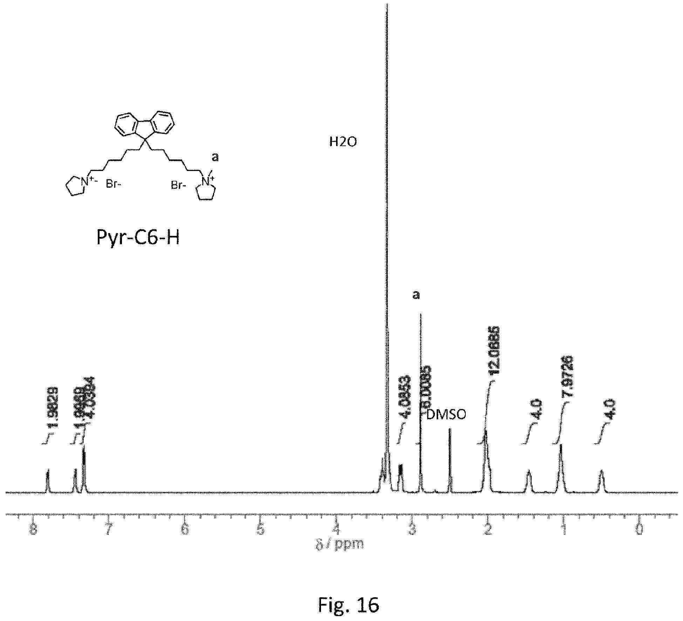

### (¹H-NMR spectrum of Pyr-C6-H (see Fig. 16))

¹H-NMR (400 MHz, DMSO-d6): 7.81 (2H, m), δ 7.44 (2H, m), δ 7.32 (4H, m), δ 3.42 (8H, m), δ 3.15 (4H, t), δ 2.88 (6H, s), δ 2.02 (12H, m), δ 1.45 (4H, m), 1.03 (8H, m), δ 0.50 (4H, m).

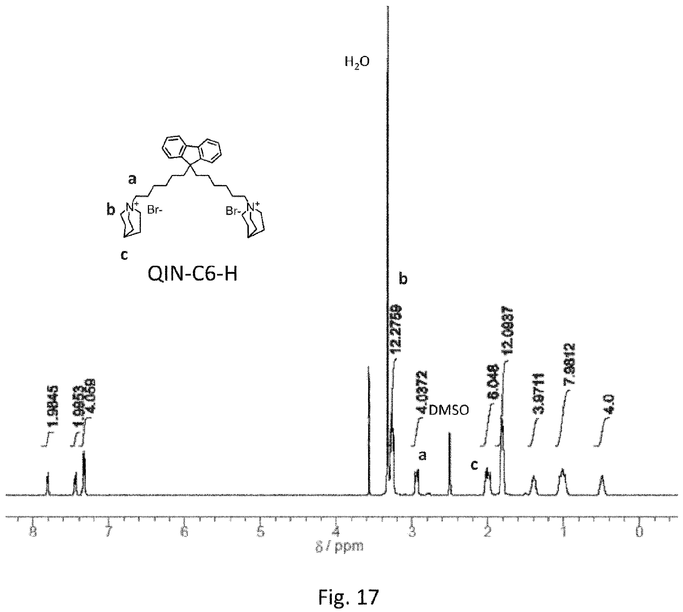

### (¹H-NMR spectrum of Qin-C6-H (see Fig. 17))

¹H-NMR (400 MHz, DMSO-d6): 7.81 (2H, m), δ 7.44 (2H, m), δ 7.32 (4H, m), δ 3.26 (12H, t), δ 2.94(4H, t), δ 2.02 (2H, t), δ 1.96 (4H, m), δ 1.80 (12H, m), δ 1.39 (4H, m), δ 1.01 (8H, m), δ 0.47 (4H, m).

### [Examples 9 to 11]

Sodium hydroxide (150 g) aqueous solution (300 mL) and n-tetrabutylammonium chloride (554 mg) were added to a two-neck flask, and the mixture was stirred under nitrogen. Fluorene (3.32 g, 20 mmol) was dissolved in 1,10-dichlorodecane (42.2 g, 200 mmol) under heating and this solution was added to the two-neck flask using a syringe. After reacting for 90 minutes under 90°C nitrogen, the solution was allowed to cool to room temperature. The organic phase was extracted with dichloromethane (300 mL) and washed with 1M hydrochloric acid (50 mL) and water (200 mL × 2). Dichloromethane was evaporated in an evaporator and unreacted 1,10-dichlorodecane was removed at 110°C under reduced pressure. The obtained residue was applied to a silica gel column (elution solvent: hexane), yielding the following target compound B (7.53 g, 14.6 mmol).

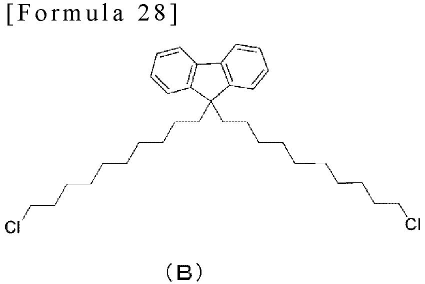

To Compound B (5.16 g, 10 mmol) was added acetone (50 ml) and sodium iodide (5 g) and allowed to react at 70°C for 12 hours. After evaporation of the solvent, it was extracted with chloroform and filtered. The residue was applied to a silica gel short column (elution solvent: hexane) and hexane was removed. To the residue, acetone (50 ml) and sodium iodide (5 g) were added again and the mixture was stirred at 70°C for 12 hours. A similar purification procedure was performed, and the compound obtained was subjected to a short column and dried in vacuo to give the following target compound C (4.26 g, 6.1 mmol).

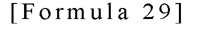

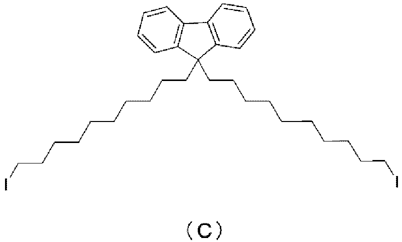

699 mg (1 mmol) of the above compound C was weighed out for each, and 5 ml of 1,4-dioxane was added. Then, each of the three amines (10 mmol) described in Examples 6 to 9 was added, and the reaction was performed at 80°C for 2 hours. 10 ml of Ethanol was then added to the mixture and allowed to react for another 2 hours. After the solvent was removed under reduced pressure, the residue was washed with hexane to give the following three compounds.

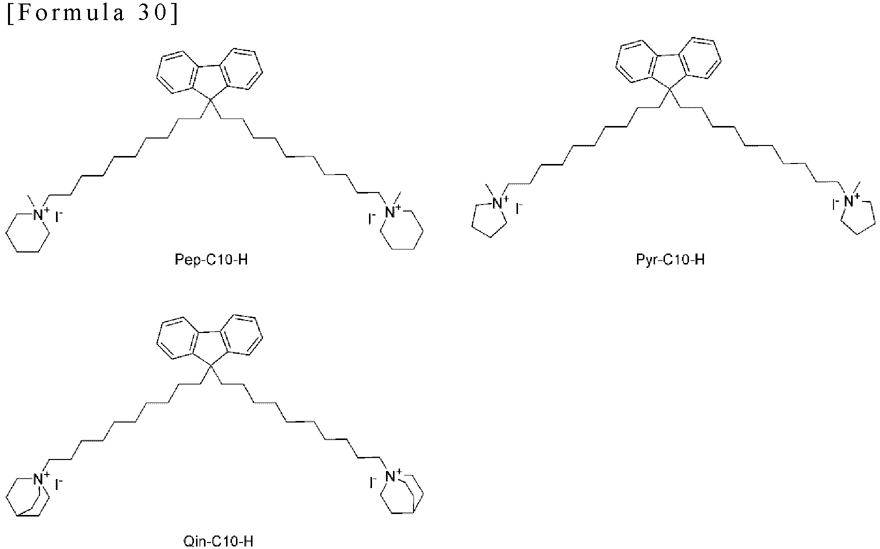

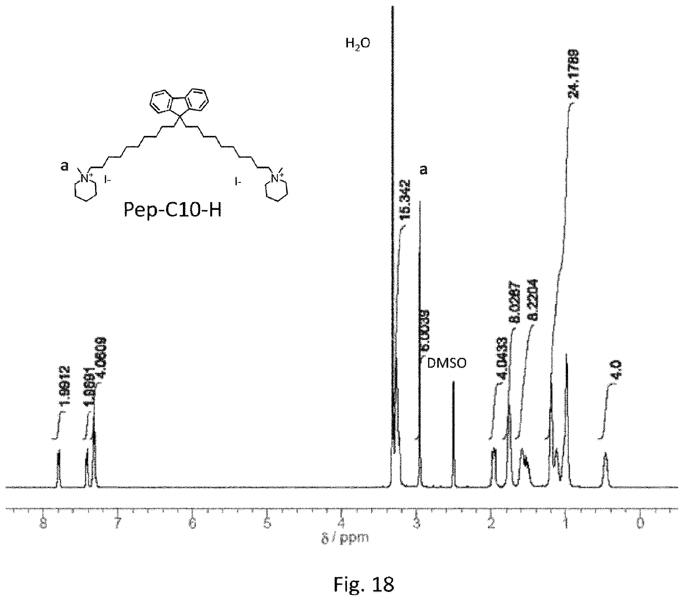

### (¹H-NMR spectrum of Pep-C10-H (see Fig. 18))

¹H-NMR (400 MHz, DMSO-d6): 7.80 (2H, m), δ 7.41 (2H, m), δ 7.32 (4H, m), δ 3.26 (12H, m), δ 2.95 (6H, s), δ 1.96 (4H, m), δ 1.76 (8H, m)δ 1.58-1.45 (8H, m), δ 1.19 (8H, m), δ 1.12 (4H, m), δ 0.99 (12H, m), δ 0.46 (4H, m).

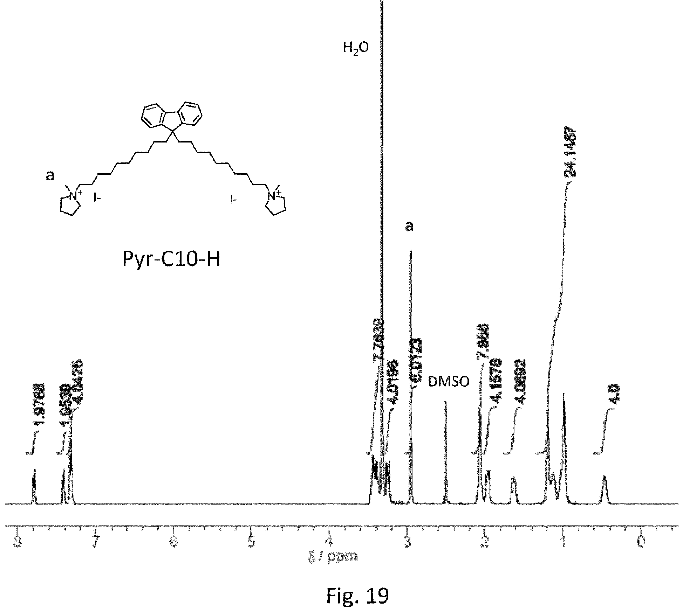

### (¹H-NMR spectrum of Pyr-C10-H (see Fig. 19))

¹H-NMR (400 MHz, DMSO-d6): 7.810 (2H, m), δ 7.42 (2H, m), δ 7.32 (4H, m), δ 3.42 (8H, m), δ 3.25 (4H, t), δ 2.95 (6H, s), δ 2.06 (8H, m), δ 1.97 (4H, m), δ 1.62 (4H, m), δ 1.19(8H, m), δ 1.12 (4H, m), δ 0.99 (12H, m), δ 0.47 (4H, m).

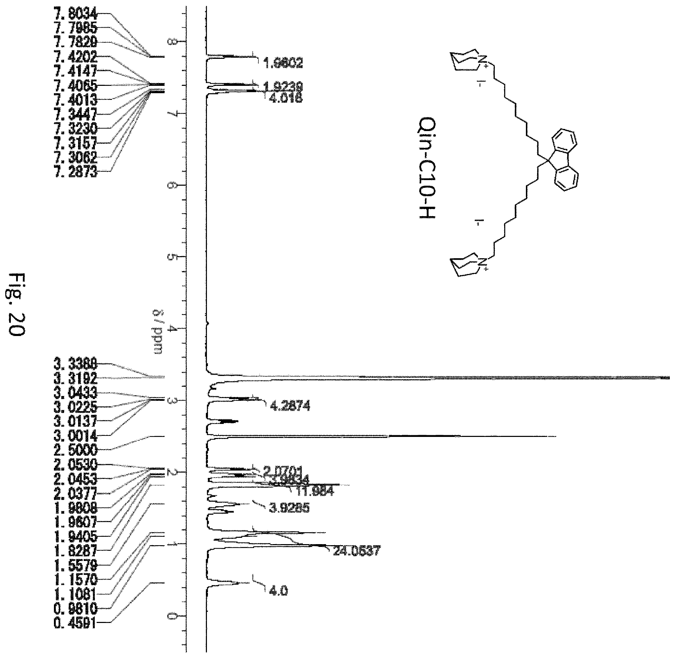

### (¹H-NMR spectrum of Qin-C10-H (see Fig. 20))

¹H-NMR (400 MHz, DMSO-d6): 7.80 (2H, d), δ 7.41 (2H, s), δ 7.31 (4H, d), δ 3.3 (12H, t), δ 3.03 (4H, t), δ 2.04 (2H, t), δ 1.96 (4H, m), δ 1.82 (12H, m), δ 1.56 (4H, m), δ 1.16 (12H, m), δ 0.98 (12H, m), δ 0.45 (4H, m).

This application claims priority to Japanese Patent Application No. 2022-164813 filed on October 13, 2022, the entire disclosure of which is incorporated by reference herein in its entirety.

## Claims

1. A compound represented by the following Formula (1):
(X¹-)₂Ar¹(-L¹-R^{N+})ₙ(A^{c-})_{n/c} ··· (1)
wherein
X¹ is a hydrogen atom or a halogen atom,
Ar¹ is a 2+n valent group having an aromatic ring,
L¹ is a single bond or divalent hydrocarbon group,
R^{N+} is a group having a ring structure containing N⁺,
A^{c-} is a c valent counter anion,
n is an integer of 1 or more, and
c is 1 or 2.

2. The compound according to claim 1, wherein the L¹ is alkylene group having 1 to 20 carbon atoms.

3. The compound according to claim 1, wherein the R^{N+} is represented by the following Formulas (N1) to (N8): 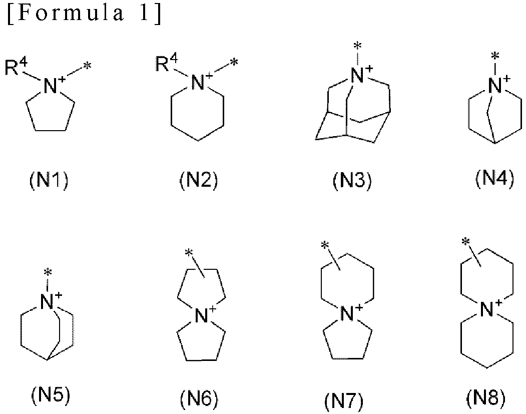 wherein
R⁴ is an alkyl group having 1 to 6 carbon atoms,
* represents a bonding hand with L¹.

4. The compound according to claim 1, wherein the (X¹-)₂Ar¹(-L¹-R^{N+})ₙ is represented by the following Formulas (a1) to (a12): 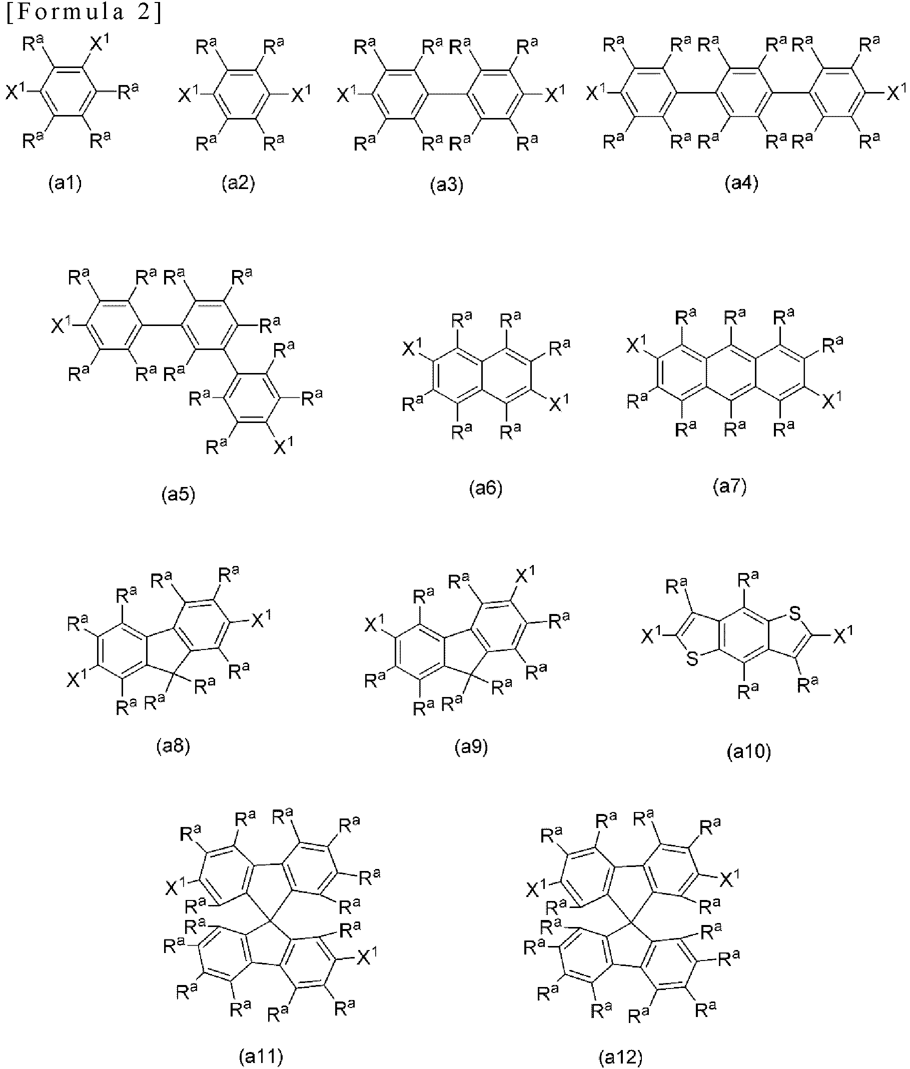 wherein
R^{a} is a hydrogen atom, a group having no ionic functional group, or L¹-R^{N+}, and at least one of R^{a} is L¹-R^{N+}.

5. A polymer having a structural unit represented by the following Formula (2): 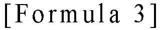 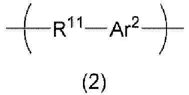 wherein
Ar² is a divalent group having an aromatic ring,
R¹¹ is a group represented by the following Formula (11),
(*-)₂Ar¹(-L¹-R^{N+})ₙ(A^{c-})_{n/c} ··· (11)
Ar¹ is a 2+n valent group having an aromatic ring,
L¹ is a single bond or divalent hydrocarbon group,
R^{N+} is a group having a ring structure containing N⁺,
A^{c-} is a c valent counter anion,
n is an integer of 1 or more,
c is 1 or 2, and
* represents a bonding hand with Ar².

6. The polymer according to claim 5, wherein the (*-)₂Ar¹(-L¹-R^{N+})ₙ is represented by the following Formulas (a21) to (a32): 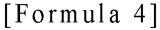 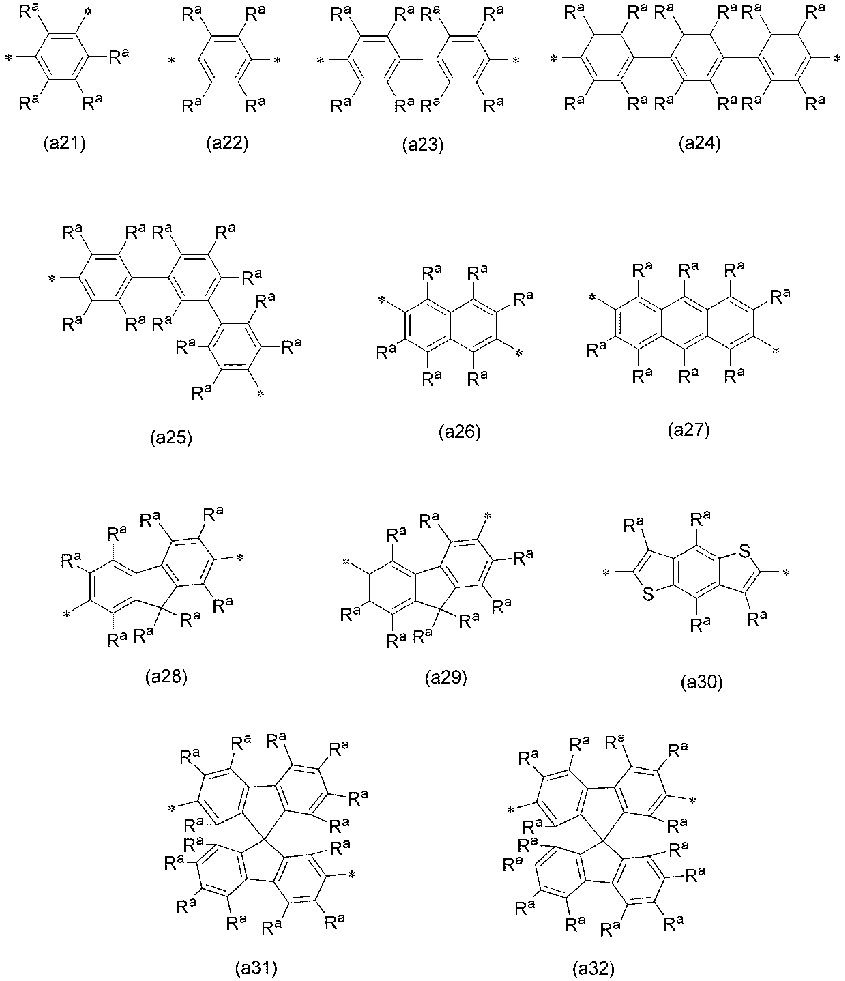 wherein
R^{a} is a hydrogen atom, a group having no ionic functional group, or L¹-R^{N+}, and at least one of R^{a} is L¹-R^{N+}, and
* represents a bonding hand with Ar².

7. An electrolyte membrane comprising the polymer according to claim 5 or 6.

8. A fuel cell comprising the electrolyte membrane according to claim 7.

9. An electrolysis apparatus comprising the electrolyte membrane according to claim 7.
